# EUROPÄISCHE PATENTSCHRIFT

(11) **EP 2 792 376 B1**
(45) Veröffentlichungstag und Bekanntmachung des Hinweises auf die Patenterteilung: **25.01.2017**
(21) Anmeldenummer: 14171391.7
(22) Anmeldetag: 09.03.2010
(51) Int. Cl.: A61M 1/14

(54) **Abdichtungseinrichtung zum Abdichten eines Volumens einer medizinischen Behandlungsanordnung gegen ein weiteres Volumen sowie Anordnung und Verfahren**
Sealing device for sealing a volume of a medical treatment assembly against another volume and assembly and method
Dispositif d'étanchéification destiné à étanchéifier un volume d'une installation de traitement médical par rapport à un autre volume, agencement et procédé

(30) Priorität: 10.03.2009 DE 102009012632
(43) Veröffentlichungstag der Anmeldung: 22.10.2014
(62) Teilanmeldung aus: 10710197.4
(73) Patentinhaber: Fresenius Medical Care Deutschland GmbH, 61352 Bad Homburg (DE)
(72) Erfinder: Günther, Götz, 61440 Oberursel (DE); Köhler, Markus, 61440 Oberursel (DE); Lapp, Uwe, 35510 Butzbach (DE); Lauer, Martin, 66606 St. Wendel (DE); Müller, Ralf, 61348 Bad Homburg (DE); Scheunert, Peter, 61381 Friedrichsdorf (DE); Schulz, Wolfgang, 66606 St. Wendel (DE); Wäber, Udo, 63071 Offenbach (DE); Weis, Manfred, 66606 St. Wendel (DE)
(74) Vertreter: Bobbert & Partner Patentanwälte PartmbB

(56) Entgegenhaltungen:
- WO-A1-01/17650
- WO-A2-2005/042065
- WO-A2-2009/006501
- DE-A1- 10 034 711
- US-A1- 2003 220 598

## Beschreibung

Die vorliegende Erfindung betrifft eine medizinische Anordnung zur Behandlung wenigstens eines medizinischen Fluids mit wenigstens einer Abdichtungseinrichtung zum Abdichten wenigstens eines ersten Volumens gegen wenigstens ein zweites Volumen gemäß dem Anspruch 1.

Bei technischen Anordnungen, wie beispielsweise Behandlungsmaschinen der Medizintechnik, labortechnischen Anordnungen oder auch Anordnungen zur Nahrungsmittelherstellung ist oftmals eine funktionelle Ankopplung externer Funktionseinrichtungen an eine Anordnung vorgesehen. Ein Beispiel einer solchen externen Funktionseinrichtung ist eine Disposable-Kassette, wie sie in der DE 10 2007 042 964 beschrieben ist. Eine medizinische Anordnung mit einer Abdichteinrichtung ist aus der US 2003/0220598 A1 bekannt. Diese offenbart eine medizinische Vorrichtung mit einer Abdichteinrichtung, einem Stößel um diese zu verformen, einem Aufnahmeabschnitt mit einer Bohrung für den Stößel und einer Luftverteilerplatte zum Zuführen von Gas in Richtung Stößel.

Eine funktionelle Ankopplung setzt ein exaktes Verbinden von einzelnen Komponenten der externen Funktionseinrichtung mit Komponenten (im Folgenden auch als Ankoppelpartner bezeichnet) der Anordnung zueinander voraus. Aufgrund von Bautoleranzen und/oder zum Ermöglichen erwünschter Ausgleichsbewegungen einzelner Bauteile im Gebrauch können dabei Öffnungen und Spalte entstehen. Diese können unerwünscht, toleriert, beabsichtigt oder sogar erforderlich sein. Durch sie hindurch können Schmutzpartikel, Keime, Fluide und dergleichen in ein Volumen - beispielsweise einen inneren Raum oder ein Inneres - der Anordnung eindringen, was unerwünscht und mit auf der Hand liegenden Nachteilen verbunden sein kann.

Aufgabe der vorliegenden Erfindung ist es, eine weitere medizinische Anordnung mit einer Abdichtungseinrichtung zum Abdichten eines ersten Volumens der Anordnung zur Behandlung medizinischer Fluide gegen ein zweites Volumen bereitzustellen. Ferner soll eine Anordnung zum Behandeln medizinischer Fluide, welche eine derartige Abdichtungseinrichtung aufweist, sowie ein Verfahren zum Behandeln medizinischer Fluide angegeben werden.

Die erfindungsgemäße Aufgabe wird durch eine erfindungsgemäße Anordnung mit einer Abdichtungseinrichtung mit den Merkmalen des Anspruchs 1 gelöst.

Ihre Abdichtungseinrichtung ist zum Abdichten wenigstens eines ersten Volumens einer Anordnung, mittels welcher bestimmungsgemäß wenigstens ein medizinisches Fluid behandelt wird, gegen wenigstens ein zweites Volumen geeignet ausgestaltet und vorgesehen.

Dazu weist die Abdichtungseinrichtung optional wenigstens eine erste Verbindungseinrichtung auf, mittels welcher die Abdichtungseinrichtung mit der Anordnung verbindbar ist.

Eine "Anordnung" im Sinne der vorliegenden Erfindung ist vorzugsweise eine medizintechnische Anordnung, wie beispielsweise eine Blutbehandlungsvorrichtung, etwa eine Dialysevorrichtung. Sie kann ferner eine Anordnung in der Labortechnik, wie Analysevorrichtungen, wie Chromatographievorrichtungen, Waagen, und dergleichen, eine Anordnung in der Arzneimittelherstellung oder dergleichen sein. Weitere Beispiele sind unten stehend genannt.

Der Begriff "medizinische Fluide" umfasst, ohne darauf beschränkt zu sein, medizinische Flüssigkeiten wie beispielsweise Blut, Substituatflüssigkeit, Sekrete und dergleichen.

Der Begriff "Abdichten", wie er hierin verwendet wird, bezeichnet das räumliche Abtrennen eines ersten Volumens von einem zweiten Volumen der Anordnung.

Von einem Abdichten im Sinne der Erfindung kann gesprochen werden, wenn die Abdichtung ein Übertreten von Fluiden, insbesondere von Gas oder Flüssigkeiten, und/oder Keimen und/oder Schmutz sowie dergleichen vom ersten Volumen in das zweite Volumen verhindert. Vorzugsweise umfasst das Abdichten auch ein Verhindern des Übertritts der vorgenannten Stoffe oder Stoffen in den vorgenannten Zuständen vom zweiten in das erste Volumen und/oder in beide Richtungen zugleich.

Ein "erstes Volumen" kann ein beliebiger Bereich innerhalb der Anordnung sein.

Das erste Volumen kann zum Aufnehmen wenigstens einer externen Funktionseinrichtung vorgesehen sein.

Das erste Volumen umfasst vorzugsweise ein Maschineninterface, also eine Ankoppelfläche oder einen Ankoppelabschnitt der Anordnung zum funktionellen Ankoppeln derselben mit der externen Funktionseinrichtung.

Das erste Volumen kann auch ein Bereich, der zumindest teilweise außerhalb oder auf der Anordnung, wie ein die Anordnung umgebender Bereich, beispielsweise die Atmosphäre, sein.

Eine "externe Funktionseinrichtung" kann vorzugsweise Energie, Messwerte und/oder Bewegungen und Kräfte auf die Anordnung übertragen oder von dieser empfangen. Sie kann in Fluidverbindung mit der Anordnung stehen. Sie kann von der Anordnung alternativ jedoch auch nur gehalten werden und mit dieser nicht in Wechselwirkung und/oder Signalkommunikation und/oder Fluidverbindung stehen.

Die "externe Funktionseinrichtung" kann ein Wärmetauscher, eine Messeinrichtung, eine multifunktionale Disposable-Kassette oder dergleichen sein. Sie kann ein Hartteil, wie beispielsweise ein Kunststoffhartteil, und eine Folie, wie beispielsweise eine Kunststofffolie, umfassen.

Eine erfindungsgemäß in Betracht gezogene externe Funktionseinrichtung kann zum Leiten medizinischer Fluide, wie Blut, Substituatflüssigkeit oder dergleichen geeignet sein. Eine derartige externe Funktionseinrichtung kann beispielsweise eine blutführende Kassette sein, sie kann Teile eines extrakorporalen Blutkreislaufs enthalten. Insbesondere kann sie als eine Disposable-Kassette ausgestaltet sein, wie sie in der DE 10 2007 042 964 beschrieben ist. Dabei gilt im Zusammenhang mit der vorliegenden Offenbarung, dass die Ausdrücke "kann aufweisen", "kann sein" und dergleichen synonym zu den hier und auch an anderer Stelle ebenfalls verwendeten Ausdrücken "weist bevorzugt auf", "ist bevorzugt" und dergleichen sind.

Das "zweite Volumen" kann in einem Inneren der Anordnung liegen. Im zweiten Volumen können sich Funktionselemente der Anordnung, wie beispielsweise Sensoren und/oder Aktoren und/oder Leitungen und/oder Teile derselben befinden.

Das zweite Volumen weist vorzugsweise einen Aufnahmeabschnitt zum Aufnehmen der externen Funktionseinrichtung auf und/oder ist durch einen derartigen Aufnahmeabschnitt begrenzt.

Das zweite Volumen kann ein Bereich im Anordnungsinneren, wie beispielsweise ein Bereich unterhalb einer beweglich oder pendelnd angeordneten und/oder um eine Achse drehbar gelagerten AS-Platte oder eines Kontaktabschnitts für die externe Funktionseinrichtung sein.

Weder das erste Volumen noch das zweite Volumen müssen begrenzte oder bestimmte Räume oder Rauminhalte sein. Sie können jedoch einzeln oder jeweils begrenzt sein oder vorbestimmte Räume sein oder Rauminhalte umfassen.

Ein "Aufnahmeabschnitt" im Sinne der vorliegenden Erfindung ist ein Abschnitt, der zum Aufnehmen wenigstens einer externen Funktionseinrichtung geeignet ist. Dabei kann Aufnehmen ein Halten, ein Umfassen, ein Umgreifen, ein Bedecken der externen Funktionseinrichtung und dergleichen sowie Kombinationen hieraus bedeuten.

Der Aufnahmeabschnitt, der flach bzw. eben sein kann, aber nicht sein muss, kann eine Wechselwirkung oder Signalkommunikation zwischen der externen Funktionseinrichtung und dem Aufnahmeabschnitt herstellen.

Der Aufnahmeabschnitt kann eine Aufnahmeplatte, wie beispielsweise eine vorzugsweise stabile "Aktor-/Sensor-Platte" (kurz "AS-Platte") oder dergleichen, sein.

Der Aufnahmeabschnitt kann beweglich, pendelnd und/oder um wenigstens eine Achse drehbar gelagert sein. Er kann so einen Verformungsausgleich erreichen.

Der Aufnahmeabschnitt kann eine angepasste Verformbarkeit aufweisen. Dies kann beispielsweise durch entsprechend ausgewählte Materialien erreicht werden. Ebenso ist es beispielsweise möglich, den Mechanismus zum Aufnehmen und Verpressen bzw. Einspannen der externen Funktionseinrichtung in bzw. mit der Anordnung so zu gestalten, dass ein Verformungsausgleich möglich ist. Dies kann zum Beispiel mit Hilfe eines Aufnahmeabschnitts zum Aufnehmen wenigstens einer externen Funktionseinrichtung erreicht werden, wie sie in der Patentanmeldung der Anmelderin der vorliegenden Erfindung beim Deutschen Patent- und Markenamt mit dem Titel "Vorrichtung zum Verbinden einer externen Funktionseinrichtung mit einer Anordnung, Anordnung aufweisend eine solche Vorrichtung und Verfahren zum Verbinden" am selben Anmeldetag wie die vorliegende Anmeldung unter der Anmeldenummer DE 10 2009 012 633.3-44 der gemeinsamen Anmelderin zum Patent eingereicht wurde, offenbart ist.

Ferner kann der Aufnahmeabschnitt als Vakuumabdichtung zwischen einem Inneren der Anordnung und der Atmosphäre und/oder einem Inneren der Anordnung und einem Behandlungsort, in dem beispielsweise eine Behandlung medizinischer Fluide erfolgt, fungieren. Ferner kann er auch geeignet ausgestaltet und vorgesehen sein, um mittels an seiner Oberfläche vorgesehenen Kanälen eine Vakuumleitung zu ermöglichen.

Eine "AS-Platte" kann Aktoren und/oder Sensoren und/oder Leitungen zum Ankoppeln an die externe Funktionseinrichtung oder Teile derselben aufweisen. Beispielsweise können eine Luftverteilerplatte und/oder ein Substituatkonnektor in und/oder an der AS-Platte vorgesehen sein.

Ebenso können Sensoren und/oder Aktoren und/oder Leitungen und/oder Teile derselben in die AS-Platte integriert bzw. gefasst sein. Es können ferner alle erfindungsgemäß eingesetzten Sensoren von der AS-Platte aufgenommen und/oder auf dieser positioniert werden.

Eine solche AS-Platte kann als Aufnahmeabschnitt zum Positionieren und Fixieren der externen Funktionseinrichtung dienen. Sie kann wie jeder Aufnahmeabschnitt im Sinne der vorliegenden Erfindung als Gegenlager zur Verpressung der externen Funktionseinrichtung dienen.

Eine derartige pendelnd und/oder um eine Achse drehbar gelagerte AS-Platte kann beispielsweise eine in der Patentanmeldung, wie sie in der Patentanmeldung der Anmelderin der vorliegenden Erfindung beim Deutschen Patent- und Markenamt mit dem Titel "Vorrichtung zum Verbinden einer externen Funktionseinrichtung mit einer Anordnung, Anordnung aufweisend eine solche Vorrichtung und Verfahren zum Verbinden" am selben Anmeldetag wie die vorliegende Anmeldung unter der Anmeldenummer DE 10 2009 012 633.3-44 der gemeinsamen Anmelderin zum Patent eingereicht wurde, beschriebene Ankoppelfläche sein.

Die Definition und/oder Rauminhalt des ersten und des zweiten Volumens ergeben sich aus der Konstruktion und/oder des aktuellen Zustands der Anordnung. In einembeispielsweise mittels einer Tür der Anordnung - geschlossenen Zustand der Anordnung können sich sowohl das erste Volumen und das zweite Volumen im Inneren der Anordnung befinden. In einem geöffneten Zustand der Anordnung-beispielsweise bei geöffneter Tür der Anordnung - kann das erste Volumen die Atmosphäre bzw. die nähere Umgebung der Anordnung sein.

Eine "Verbindungseinrichtung" im Sinne der vorliegenden Erfindung bezeichnet eine Einrichtung, die zum Verbinden der Abdichtungseinrichtung mit der Anordnung geeignet ausgestaltet und vorgesehen ist.

Die erste Verbindungseinrichtung kann dabei integraler Teil der Abdichtungseinrichtung sein. Sie kann jedoch auch eine mit der Abdichtungseinrichtung form- und/oder kraftschlüssig verbundene Verbindungseinrichtung sein.

Die erste Verbindungseinrichtung kann an wenigstens einem Abschnitt einer Seite der Abdichtungseinrichtung vorhanden sein. Sie kann jedoch auch an einer Oberfläche und/oder an einer Unterseite der Abdichtungseinrichtung vorgesehen oder mit dieser verbunden sein.

Erfindungsgemäß kann jede Verbindungseinrichtung aus einem oder mehreren Verbindungselementen ausgestaltet sein.

Der Begriff "Verbinden" oder "Herstellen einer Verbindung" wie er hierin verwendet wird, kann ein funktionelles und/oder mechanisches Verbinden der externen Funktionseinrichtung mit der Anordnung bezeichnen oder umfassen. Das "Verbinden" von zwei Elementen miteinander führt zu einer abdichtenden Verbindung im Bereich der Verbindungseinrichtung im Sinne des hierin verwendeten Begriffs "Abdichten".

Eine geeignete Verbindung zwischen der Abdichtungseinrichtung und der Anordnung kann eine kraft- und/oder form- und/oder stoffschlüssige Verbindung sein.

Die Abdichtungseinrichtung kann mit wenigstens einem Abschnitt der Anordnung, wie beispielsweise einer Trägereinrichtung zum Aufnehmen der AS-Platte in einem Inneren der Anordnung, und/oder mit der AS-Platte und/oder einem anderen geeigneten Element der Anordnung verbunden werden.

Eine derartige "Trägereinrichtung" kann eine stabile Trägereinrichtung, wie beispielsweise ein Trägerrahmen sein.

Der Trägerrahmen kann Teil der Aufnahmeeinrichtung zum Aufnehmen der wenigstens einen externen Funktionseinrichtung sein.

Die Trägereinrichtung kann kraft- und/oder form- und/oder stoffschlüssig mit der Anordnung verbunden sein.

Durch die zwischen der Abdichtungseinrichtung und einer Trägereinrichtung geschaffene Verbindung kann ebenfalls ein Abdichten des ersten Volumens der Anordnung gegenüber dem zweiten Volumen erreicht werden.

Vorteilhafte Weiterentwicklungen der vorliegenden Erfindung sind jeweils Gegenstand der Unteransprüche.

In einer bevorzugten Ausführungsform der vorliegenden Erfindung ist die erste Verbindungseinrichtung zur Verbindung mit einer zweiten Verbindungseinrichtung ausgestaltet, welche funktionell oder strukturell Teil der Anordnung ist.

Eine "zweite Verbindungseinrichtung", wie sie erfindungsgemäß eingesetzt werden kann, kann ausgestaltet sein, um eine kraft- und/oder form- und/oder stoffschlüssige Verbindung mit der ersten Verbindungseinrichtung einzugehen.

Die Anzahl der Elemente zweiten Verbindungseinrichtungen kann der Anzahl der Elemente, aus welchen die erste Verbindungseinrichtung ausgestaltet ist, entsprechen. Des Weiteren kann die Position, d.h. räumliche Anordnung, der zweiten Verbindungseinrichtung an der Anordnung so gewählt werden, dass sie der ersten Verbindungseinrichtung der Abdichtungseinrichtung gegenüberliegt.

Die Geometrie und/oder Abmessung der zweiten Verbindungseinrichtung kann dabei derart vorgesehen und ausgestaltet sein, dass sie in einer Art "Schlüssel-Schloss-Prinzip" mit der ersten Verbindungseinrichtung kombinierbar bzw. verbindbar ist. Die erste und die zweite Verbindungseinrichtung können entsprechend ein Verbindungspaar bilden. Dies erleichtert ein Verbinden der ersten Verbindungseinrichtung mit der zweiten Verbindungseinrichtung (oder umgekehrt) und vereinfacht damit den Vorgang des Verbindens. Zudem kann oftmals eine verbesserte Abdichtung erzielt werden. Ferner kann es möglich sein, ein sicheres Verbinden und damit ein zuverlässiges Abdichten ebenso wie Fehler beim Verbinden oder Abdichten einfacher zu erkennen.

Die zweite Verbindungseinrichtung kann kraft- und/oder form- und/oder stoffschlüssig mit der Anordnung verbunden sein. Sie kann einen Teil der Anordnung bilden. Sie kann ebenso lösbar mit der Anordnung verbunden sein.

In einer weiteren Ausführungsform der vorliegenden Erfindung kann die erste Verbindungseinrichtung zur kraft- und/oder formschlüssigen Verbindung mit der zweiten Verbindungseinrichtung ausgestaltet sein.

Geeignete kraftschlüssige Verbindungen schließen durch Druck- und/oder Reibungskräfte erwirkte Verbindungen ein. Sie schließen, ohne darauf beschränkt zu sein, durch Schrauben, Keilen, Klemmen und/oder unter Verwenden einer Federklemme erzeugte Verbindungen ein.

Geeignete formschlüssige Verbindungen schließen durch Ineinandergreifen der ersten und der zweiten Verbindungseinrichtung erzeugte Verbindungen ein. Sie schließen, ohne darauf beschränkt zu sein, lösbare Verbindungen, wie Schnappverbindungen, Nut-FederVerbindungen, Spundungen, Schwalbenschwanzverbindungen, Verbindungen mittels Passfedern, sowie nicht lösbare Verbindungen, wie beispielsweise durch Vernietung, Verstiften oder Verwenden von Klebeankern, erzeugte Verbindungen ein.

Die erste Verbindungseinrichtung kann vorzugsweise passgenau in die Aufnahmeeinrichtung eingeführt werden.

Die erste Verbindungseinrichtung kann eine Verdickung und/oder einen Widerhaken oder ein anderes geeignetes Element aufweisen, mittels derer es in der Aufnahmeeinrichtung verhakt werden kann.

Ein solches "verhakendes Element" kann an der ersten Verbindungseinrichtung vorgesehen sein, die erste Verbindungseinrichtung kann jedoch ebenso selbst in Form eines solchen Elements ausgestaltet sein. Ebenso kann die zweite Verbindungseinrichtung mit einem "verhakenden Element" oder als solches ausgestaltet sein.

Die Verbindung zwischen der Abdichtungseinrichtung und der Anordnung kann eine leicht lösbare Verbindung sein, die zum Beispiel durch bloßes Herausziehen der ersten

Verbindungseinrichtung aus der zweiten Verbindungseinrichtung - beispielsweise durch Zug an der Abdichtungseinrichtung - gelöst werden kann. Sie kann auch eine nur unter nennenswertem Kraftaufwand lösbare Verbindung sein.

Wenn mehrere Elemente jeweils der ersten und zweiten Verbindungseinrichtung miteinander verbunden werden sollen, können die entsprechenden Verbindungspaare auf gleiche oder verschiedene Art miteinander verbunden werden.

In einer weiteren bevorzugten Ausführungsform der vorliegenden Erfindung ist die erste Verbindungseinrichtung als einsteckbare Einrichtung ausgestaltet, welche in die als eine Aufnahmeeinrichtung für die einsteckbare Einrichtung ausgestaltete zweite Verbindungseinrichtung einsteckbar ist.

Die erste Verbindungseinrichtung kann an wenigstens einer Rand- oder Oberfläche der Abdichtungseinrichtung vorstehen.

Die zweite Verbindungseinrichtung, welche eine Aufnahmeeinrichtung für die erste Verbindungseinrichtung bildet, kann eine in der Anordnung vorgesehene Einrückung und/oder Einkerbung sein oder aufweisen.

Die Aufnahmeeinrichtung kann die einsteckbare Einrichtung umfassen und/oder umgreifen.

Die einsteckbare Einrichtung kann vorzugsweise passgenau in die Aufnahmeeinrichtung eingesteckt werden. Die einsteckbare Einrichtung kann eine Verdickung und/oder einen Widerhaken oder ein anderes geeignetes Element aufweisen, mittels welchem es in der Aufnahmeeinrichtung verhakt werden kann.

Die einsteckbare Einrichtung kann ein größeres Volumen als das aufnehmende Volumen der Aufnahmeeinrichtung aufweisen. Sie kann jedoch auch ein kleineres Volumen aufweisen und ein Volumen in der Aufnahmeeinrichtung freilassen. Im letzt genannten Fall kann die einsteckbare Einrichtung innerhalb der Aufnahmeeinrichtung bewegbar sein. Daher kann die Abdichtungseinrichtung in einem solchen Fall zu einer erhöhten Beweglichkeit zwischen Anordnung und Abdichtungseinrichtung oder Ankoppelfläche bzw. AS-Fläche beitragen.

In einer weiteren bevorzugten Ausführungsform der vorliegenden Erfindung ist die zweite Verbindungseinrichtung als einsteckbare Einrichtung ausgestaltet, welche in die als eine Aufnahmeeinrichtung für die einsteckbare Einrichtung ausgestaltete erste Verbindungseinrichtung einsteckbar ist.

Da eine solche Ausführungsform der vorliegenden Erfindung eine Umkehrung der vorstehend beschriebenen Ausführungsform darstellt, kann zur Beschreibung der vorliegenden Ausführungsform - unter Berücksichtigung des Austauschs von erster und zweiter Verbindungseinrichtung - auf die vorstehend angegebene Beschreibung verwiesen werden.

In einer weiter bevorzugten Ausführungsform der vorliegenden Erfindung weist die Abdichtungseinrichtung ferner eine "dritte Verbindungseinrichtung" zur Verbindung der Abdichtungseinrichtung mit einem Aufnahmeabschnitt der Anordnung oder einer Aufnahmeeinrichtung zum Aufnehmen der externen Funktionseinrichtung auf.

Des Weiteren kann die dritte Verbindungseinrichtung ausgestaltet sein, um mit einer vierten Verbindungseinrichtung des Aufnahmeabschnitts verbindbar zu sein.

Die dritte Verbindungseinrichtung und die vierte Verbindungseinrichtung können sich auf einander zugewandten Seiten der Abdichtungseinrichtung und des Aufnahmeabschnitts gegenüberliegen. Beispielsweise kann die dritte Verbindungseinrichtung an einer Unterseite der Abdichtungseinrichtung vorgesehen sein. Die vierte Verbindungseinrichtung kann entsprechend an einer Oberfläche des Aufnahmeabschnitts angeordnet sein.

Die dritte Verbindungseinrichtung kann, wie vorstehend ausgeführt, als einsteckbare Einrichtung oder als Aufnahmeeinrichtung für eine einsteckbare Einrichtung ausgeführt sein. Zur Beschreibung einer derart ausgestalteten dritten Verbindungseinrichtung wird an dieser Stelle auf die vorstehend beschriebenen Ausführungsformen verwiesen.

Dementsprechend kann die "vierte Verbindungseinrichtung" als Aufnahmeeinrichtung für eine einsteckbare Einrichtung oder als einsteckbare Einrichtung ausgestaltet sein.

Die dritte und die vierte Verbindungseinrichtung können entsprechend ein Verbindungspaar bilden.

In einer weiter bevorzugten Ausführungsform sind wenigstens die erste Verbindungseinrichtung, die zweite Verbindungseinrichtung, die dritte Verbindungseinrichtung und/oder die vierte Verbindungseinrichtung umlaufend ausgestaltet.

Der Begriff "umlaufend", wie er hierin verwendet wird, bedeutet, dass die betreffende (erste, zweite, dritte und/oder vierte) Verbindungseinrichtung geschlossen, d.h. nicht oder im Wesentlichen nicht unterbrochen um einen von ihr umfassten Bereich herum ausgestaltet ist.

Die Verbindungseinrichtung kann dabei an einem äußeren Rand der Abdichtungseinrichtung umlaufend vorgesehen sein. Sie kann an einem inneren Rand eines Bauteils der Anordnung umlaufend vorgesehen sein. Sie kann beispielsweise am inneren Rand der Trägereinrichtung umlaufend vorgesehen sein. Sie kann ebenso in wenigstens einem Abschnitt der Unterseite der Abdichtungseinrichtung vorgesehen sein. Sie kann ferner in wenigstens einem Abschnitt der Oberfläche des Aufnahmeabschnitts zum Aufnehmen der externen Funktionseinrichtung vorgesehen sein.

In einer weiter bevorzugten Ausführungsform sind eine oder mehrere der Verbindungseinrichtungen als Dichtlippen ausgestaltet. Die Dichtlippen können eine elastische Dichtung darstellen und beispielsweise aus Metall, Kunststoff, elastischem Gummi oder Kombinationen hiervon ausgestaltet sein.

In einer weiter bevorzugten Ausführungsform der vorliegenden Erfindung sind die erste Verbindungseinrichtung zusammen mit der zweiten Verbindungseinrichtung und/oder die dritte Verbindungseinrichtung zusammen mit der vierten Verbindungseinrichtung ausgestaltet, um jeweils gemeinsam eine fluiddichte Abdichtung zu bewirken.

Der Begriff "fluiddicht", wie er hierin verwendet wird, gibt an, dass das Eindringen eines beliebigen Fluids und/oder das Durchdringen eines Bauteils mit einem beliebigen, beispielsweise einem medizinischen Fluid, wie Blut, Substituatflüssigkeit oder dergleichen, im Wesentlichen verhindert wird.

Eine fluiddichte Abdichtung verhindert vorzugsweise auch das Eindringen von Gasen, wie beispielsweise Luft, aus dem ersten Volumen in das zweite Volumen oder umgekehrt.

In einer weiter bevorzugten Ausführungsform weist die erste Verbindungseinrichtung in wenigstens einem Abschnitt eine geringere Dicke auf als ein weiterer Abschnitt der Abdichtungseinrichtung.

Aufgrund eines geringeren Dicken ist ein derartiger Abschnitt der ersten Verbindungseinrichtung bei gleichem Material flexibler ausgestaltbar. Die Abdichtungseinrichtung kann entsprechend beweglich ausgestaltet sein.

Der Abschnitt mit geringerer Dicke kann in der Nähe der ersten Verbindungseinrichtung und/oder zwischen der ersten und der dritten Verbindungseinrichtung angeordnet sein. Er kann ferner derjenige Abschnitt der Abdichtungseinrichtung sein, welcher einen Freiraum zwischen dem Aufnahmeabschnitt zum Aufnehmen einer externen Funktionseinrichtung und der Trägereinrichtung überdeckt.

Beispielsweise weist die Abdichtungseinrichtung - vorzugsweise zwischen zwei umlaufenden Dichtlippen - einen umlaufenden Bereich mit geringerer Dicke der Abdichtungseinrichtung auf. Eine derart ausgestaltete Abdichtungseinrichtung kann zum Beispiel während eines Vorgangs des Ankoppelns einer externen Funktionseinrichtung an der Anordnung einer Bewegung des Aufnahmeabschnitts in dem Bereich der geringeren Dicke der Abdichtungseinrichtung lokal nachgeben. Ein solcher Bereich von geringerer Dicke kann zum Beispiel ein Bereich in unmittelbarer Nähe der Öffnung bzw. des Spalts zwischen dem Aufnahmeabschnitt und der Trägereinrichtung angeordnet sein. Eine solche geringere Dicke kann vorteilhafter Weise beispielsweise ein Schwenken der Abdichtungseinrichtung oder eines Bereichs hiervon, insbesondere eines Randbereichs, um beispielsweise drei Grad bezogen auf eine Ruhe- oder Haupterstreckung, zulassen.

In einer weiter bevorzugten Ausführungsform der vorliegenden Erfindung bedeckt die Abdichtungseinrichtung wenigstens einen Abschnitt einer Oberfläche des Aufnahmeabschnitts zum Aufnehmen der externen Funktionseinrichtung.

Eine solche "Oberfläche des Aufnahmeabschnitts" kann die der externen Funktionseinrichtung zugewandte Fläche sein. Sie kann die Seite sein, auf der die externe Funktionseinrichtung ruht.

Die Abdichtungseinrichtung kann die Oberfläche des Aufnahmeabschnitts in einem Randbereich oder mehreren Randbereichen des Aufnahmeabschnitts bedecken. Sie kann einen inneren Bereich oder einen Mittelbereich des Aufnahmeabschnitts freilassen. Die Abdichtungseinrichtung kann den Aufnahmeabschnitt in vorbestimmten Bereichen bedecken.

In einer weiter bevorzugten Ausführungsform bedeckt die Abdichtungseinrichtung in ihrem Gebrauchszustand die gesamte Oberfläche des Aufnahmeabschnitts. Dabei kann die Oberfläche im Rahmen der vorliegenden Erfindung als die zur externen Funktionseinrichtung zugewandte Fläche zu verstehen sein, also beispielsweise die Fläche, auf welcher die externe Funktionseinrichtung ruht.

Die Abdichtungseinrichtung kann beispielsweise die beweglich gelagerte AS-Platte bzw. Ankoppelfläche der externen Funktionseinrichtung überspannen und gleichzeitig den Spalt bzw. die Öffnung zwischen der beweglich gelagerten AS-Platte und der umgebenden feststehenden Trägereinrichtung, beispielsweise einem Maschinenrahmen, überspannen und abdichten.

Die Abdichtungseinrichtung kann die Oberfläche des Aufnahmeabschnitts gleichmäßig bedecken. Sie kann eine in jedem Bereich gleich große Dicke aufweisen. Sie kann ferner Bereiche mit stärkerer oder geringerer Dicke aufweisen.

In einer weiter bevorzugten Ausführungsform der vorliegenden Erfindung ist die Abdichtungseinrichtung ausgestaltet und vorgesehen, um kraft- und/oder formschlüssig mit einem Aufnahmeabschnitt der Vorrichtung zum Aufnehmen der externen Funktionseinrichtung verbindbar zu sein.

Die Abdichtungseinrichtung kann beispielsweise durch Miteinander-Kombinieren, insbesondere Ineinanderstecken, der dritten Verbindungseinrichtung mit der vierten Verbindungseinrichtung mit dem Aufnahmeabschnitt verbindbar sein. Sie kann ferner auf den Aufnahmeabschnitt aufgeschnappt werden. Eine aufgeschnappte Abdichtungseinrichtung kann in vorteilhafter Weise schnell, einfach und leicht lösbar von dem Aufnahmeabschnitt entfernt, d.h. abgenommen, werden.

In einer weiteren bevorzugten Ausführungsform der vorliegenden Erfindung weist die dritte Verbindungseinrichtung oder die vierte Verbindungseinrichtung wenigstens eine Noppe auf.

Eine "Noppe" im Sinne der vorliegenden Erfindung bezeichnet eine Ausstülpung an der Oberfläche der dritten oder vierten Verbindungseinrichtung. Die dritte oder vierte Verbindungseinrichtung kann indessen auch selbst als Noppe ausgestaltet sein.

Eine Noppe ist vorzugsweise aus Gummi ausgestaltet.

Eine Noppe kann eine einfach auszugestaltende Verbindungseinrichtung darstellen. Die Noppe kann daneben eine leicht und schnell lösbare Verbindung darstellen.

In einer weiter bevorzugten Ausführungsform der vorliegenden Erfindung ist die Abdichtungseinrichtung ausgestaltet, um durch Aufbringen von Unterdruck mit der Oberfläche des Aufnahmeabschnitts verbindbar zu sein.

Ein solcher Unterdruck kann beispielsweise durch Ansaugen der Abdichtungseinrichtung in Richtung auf die Oberfläche des Aufnahmeabschnitts durch Öffnungen in der Abdichtungseinrichtung und/oder im Aufnahmeabschnitt und/oder durch Evakuieren eines Zwischenraums zwischen der Abdichtungseinrichtung und dem Aufnahmeabschnitt erzeugt werden.

Zum Absaugen des Zwischenraums zwischen der Abdichtungseinrichtung und dem Aufnahmeabschnitt können Luftkanäle vorgesehen sein, welche nicht von der Abdichtungseinrichtung abgedeckt oder verlegt werden. Auf diese Weise kann eine zuverlässige Luftleitung gewährleistet sein.

Ferner können ebenso eine luftleitende Zwischenschicht, wie beispielsweise ein Vlies vorgesehen, und/oder ausreichend viele Vakuum-Einleitestellen im Aufnahmeabschnitt realisiert sein.

Durch Aufbringen von Unterdruck zum Verbinden der Abdichtungseinrichtung mit der Oberfläche des Aufnahmeabschnitts kann die Abdichtungseinrichtung lösbar mit dem Aufnahmeabschnitt verbunden sein.

In einer weiter bevorzugten Ausführungsform kann die Abdichtungseinrichtung ausgestaltet sein, um unlösbar mit der Anordnung verbunden zu werden.

Eine derart unlösbare Verbindung zwischen der Abdichtungseinrichtung und der Anordnung kann durch Verkleben der Abdichtungseinrichtung mit der Anordnung, Aufvulkanisieren der Abdichtungseinrichtung an der Anordnung, Anlöten wenigstens eines Abschnitts der Abdichtungseinrichtung an der Anordnung oder auf vielerlei andere bekannte Weisen erreicht werden.

Wenn im Zusammenhang mit der vorliegenden Erfindung die Rede von einer unlösbaren Verbindung ist, so kann hierunter eine solche Abdichtungseinrichtung zu verstehen sein, welche nicht zerstörungsfrei von der Auflagefläche oder dem Aufnahmeabschnitt, z.B. der AS-Platte, entfernt, d.h. abgelöst oder abgenommen werden kann.

Eine unlösbare Verbindung der Abdichtungseinrichtung mit der Anordnung kann beispielsweise auch als so genannter "Originalitätsverschluss" realisiert sein. Ein solcher Originalitätsverschluss kann beispielsweise an der äußeren Dichtlippe vorgesehen sein. Er kann beispielsweise dazu dienen, einen Versuch des Abnehmens der Abdichtungseinrichtung zu verhindern oder - falls doch erfolgt - anzuzeigen. Etwaige Undichtigkeiten oder eine nicht mehr gewährte Abdichtung können so vorteilhaft rasch und sicher erkannt werden.

Während des Abnehmens kann hierzu die Dichtung in der Dichtungsrille der Trägereinrichtung abreißen und die Abdichtungseinrichtung auf diese Weise unbrauchbar gemacht werden.

Die Abdichtungseinrichtung kann unlösbar mit der Aufnahmeeinrichtung, z.B. in Form einer Aktor-Sensor-Platte, und/oder mit dem anordnungsseitigen Rahmen oder einem anderen Abschnitt hiervon verbunden sein. In solchen Ausführungen ist es nicht möglich, die Abdichtungseinrichtung zerstörungsfrei und/oder ohne Spezialwerkzeug, welches z.B. üblicherweise nur dem Servicetechniker vorliegt, zu entfernen oder von der Aufnahmeeinrichtung zu lösen.

In bestimmten Ausführungsformen der vorliegenden Erfindung kann die Abdichtungseinrichtung z.B. zum Zwecke des Originalitätsverschlusses, mittels mechanischer Elemente wie z.B. Haken von hinten, also von der Maschinenseite her, verhakt werden. Entsprechende Haken können vorgesehen sein, z.B. in einem Abschnitt der Aufnahmeeinrichtung. Für die Aufnahme der Haken kann die Abdichtungseinrichtung auf ihrer Rückseite beispielsweise eine umlaufende Lippe (von z.B. 10 mm Breite) aufweisen. Dabei können in der Lippe Öffnungen zum Einhaken der Haken vorgesehen sein. Diese Haken können zum Beispiel nur mit Spezialwerkzeug durch den Servicetechniker zerstörungsfrei demontierbar sein.

Haken sind dabei nur ein Beispiel eines - insbesondere mechanischen- Rückhaltesystems. Dieses kann beliebig ausgestaltet sein.

In einer weiter bevorzugten Ausführungsform weist die Abdichtungseinrichtung in wenigstens einem Abschnitt vorverformte Bereiche auf, die durch Verschwächungen oder Verstärkungen gegenüber einer Dicke der die vorverformten Bereiche umgebenden Bereiche der Abdichtungseinrichtung ausgestaltet sind.

Die Abdichtungseinrichtung kann selbst als Sensor und/oder Aktor fungieren. Dies kann beispielsweise durch Ausbilden der vorstehend genannten vorverformten Bereiche erreicht werden.

Vorverformte Bereiche der Abdichtungseinrichtung können zum Beispiel eine Sensorfunktion zur Erkennung der Anwesenheit der externen Funktionseinrichtung erfüllen.

Es kann beispielsweise gewünscht sein, durch Andrücken bzw. Abheben einer Folie der externen Funktionseinrichtung gegen das Hartteil oder den Körper der externen Funktionseinrichtung Kanäle in der externen Funktionseinrichtung fluidisch voneinander zu trennen. Dies kann beispielsweise mittels einer Kraft erreicht werden, die durch die Abdichtungseinrichtung und/oder einem in der Abdichtungseinrichtung vollständig oder zum Teil enthaltenen Aktor und/oder einem an einer AS-Platte der Anordnung vorgesehenen oder integrierten Aktor vermittelt wird. Andererseits können durch Anheben oder Andrücken der Folie solche Kanäle auch miteinander verbunden werden.

Die Abdichtungseinrichtung kann dazu Kanalöffnungen aufweisen, welche entweder permanent an Zugabestellen, zum Beispiel für Substituatflüssigkeit, und/oder schaltbar an Zugabestellen wie Substituatleitungen angeordnet sind. Die Abdichtungseinrichtung kann hierzu ferner SingleNeedle- (SN-) Kammeröffnungen aufweisen.

Die zum Andrücken bzw. Abheben der Folie notwendigen Kräfte können beispielsweise mittels der Abdichtungseinrichtung auf die Folie übertragen werden.

In einer weiter bevorzugten Ausführungsform der vorliegenden Erfindung sind die vorverformten Bereiche ausgestaltet, um eine Ventilfunktion zu erfüllen.

Eine Ventilfunktion kann beispielsweise durch einen im Folgenden beschriebenen Phantomventil-Aktor ausgeübt werden. Dieser kann vorzugsweise als Komplett-Kolbendichtung ausgestaltet sein.

Der Phantomventil-Aktor kann als mit in eine Dichtmatte integrierten Membran-Aktoren ausgestaltet sein. Die Dichtmatte kann als eine Luftverteilerplatte (kurz: LVP) ausgestaltet sein.

Dabei kann die in die LVP-Dichtmatte integrierte Aktor-Membran ausgestaltet sein, um sich unter Druck auszudehnen und den Stößel in Richtung zu einer Oberfläche der Abdichtungseinrichtung, die der externen Funktionseinrichtung an- oder gegenüberliegt, zu drücken.

Der Stößel wird pneumatisch betrieben.

Der Stößel kann eine Kolbendichtung aufweisen oder als eine solche wirken.

Der Stößel kann an der AS-Platte vorgesehen und/oder in dieser integriert sein.

Der Stößel kann geeignet ausgestaltet und vorgesehen sein, um gegen die Abdichtungseinrichtung und so gegen die Folie der externen Funktionseinrichtung zu drücken.

Der Stößel kann ebenso geeignet ausgestaltet und vorgesehen sein, um die Abdichtungseinrichtung von der Folie abzuheben. Dies kann beispielsweise aufgrund seiner Verbindung mit der AS-Platte, wie beispielsweise einer Verbindung mittels Kleben, Vulkanisieren, Aufbringen von Unterdruck und dergleichen, erreicht werden. Die Folie kann dabei vom Hartteil der externen Funktionseinrichtung abheben.

Ferner kann eine solche Funktion mit Hilfe der in der Abdichtungseinrichtung vorgesehenen vorverformten Bereiche und die Rückstellkräfte einer elastischen Abdichtungseinrichtung unterstützt werden.

In bestimmten Ausführungsformen ist die Abdichtungseinrichtung dazu ausgelegt und vorgesehen, derart auf eine Ventileinrichtung einer externen Funktionseinrichtung einwirken zu können, dass diese während des Ankoppelns automatisch und/oder vorzugsweise ohne weiteres Zutun allein durch das

Zusammenbringen von Abdichtungseinrichtung und Ventileinrichtung, in einen Gebrauchszustand verbracht werden kann.

Die Ventileinrichtung kann beispielsweise eine Ventileinrichtung einer externen Funktionseinrichtung sein, wie sie beispielsweise in der von der Anmelderin der vorliegenden Erfindung beim Deutschen Patent- und Markenamt hinterlegten Anmeldung DE 10 2009 024 469.7-44 beschrieben ist. Die externe Funktionseinrichtung kann beispielsweise den von der Anmelderin der vorliegenden Erfindung beim Deutschen Patent- und Markenamt hinterlegten Anmeldungen DE 10 2009 024 664.6 und DE 10 2009 468.9-41 entnommen werden.

Ein Ventileinsatz einer Ventileinrichtung, z.B. eines Multifunktionsventils wie in der DE 10 2009 024 469.7-44 beschrieben, befindet sich beim Sterilisieren der externen Funktionseinrichtung normalerweise in einer Position, in der der Ventileinsatz nicht vollständig bis an den Anschlag in das Hartteil einer zum Beispiel als Disposable-Kassette ausgestalteten externen Funktionseinrichtung hineingedrückt ist.

Dabei kann in bestimmten erfindungsgemäßen Ausführungsformen in dieser Stellung Sterilisiermedium, z.B. Heißdampf, Gas, z.B. Ethylenoxid (EO) in das Innere der externen Funktionseinrichtung eindringen.

In diesem Zustand verbleibt die Ventileinrichtung der fertig sterilisierten und verpackten externen Funktionseinrichtung in manchen erfindungsgemäßen Ausführungsformen im Allgemeinen auch im Auslieferungszustand an den Kunden.

Das Ankoppeln der externen Funktionseinrichtung an die Anordnung kann zum Beispiel mit Hilfe einer Maschinentür, wie sie beispielsweise aus der von der Anmelderin der vorliegenden Erfindung beim Deutschen Patent- und Markenamt hinterlegten Anmeldung DE 10 2009 012 633.3-44 hervorgeht, erfolgen.

Während des Ankoppelns der externen Funktionseinrichtung an die Anordnung zu deren Gebrauch wird in bestimmten erfindungsgemäßen Ausführungsformen der Ventileinsatz mittels einer Erhebung oder eines Vorsprungs oder dergleichen auf oder an der Oberfläche der Abdichtungseinrichtung automatisch bis zum Anschlag in das Ventilgehäuse (z.B. in den Hartteil der Disposable-Kassette) hineingedrückt.

Auf diese Weise kann in manchen erfindungsgemäßen Ausführungsformen zum Beispiel die Funktion der Ventileinrichtung als Rückschlagventil aktiviert werden, insbesondere bei Zusammenbringen von Ventileinrichtung und Abdichtungseinrichtung.

Die Erhebung auf der Oberfläche der Abdichtungseinrichtung kann während der Fertigung z.B. als Noppe (Werkstofferhebung oder -verdickung) ausgeführt oder bewirkt werden bzw. sein.

Zum Zwecke der Reinigung der Abdichtungseinrichtung im Klinikbetrieb kann vorteilhaft alternativ und/oder ergänzend hierzu vorgesehen sein, eine Erhebung wie einen Bolzen, zum Beispiel einen metallischen Bolzen, in dem Aufnahmeabschnitt vorzusehen, z.B. in einer Position genau gegenüber der Ventileinrichtung der anzukoppelnden externen Funktionseinrichtung.

Ein solcher Bolzen oder eine solche Erhebung kann ein über die Ankoppelfläche des Aufnahmeabschnitts herausragender metallischer Bolzen sein. Er kann in den Aufnahmeabschnitt eingepresst oder eingeschraubt sein. In einem nicht angekoppelten Zustand kann der Bolzen die Abdichtungseinrichtung im Wesentlichen lokal, vorzugsweise um ein definiertes Maß, z.B. 1 mm in einem Maximum der Erhebung, von dem Aufnahmeabschnitt abheben oder diese entsprechend vorstehen lassen.

So kann es vorteilhaft möglich sein, die Oberfläche der Abdichtungseinrichtung zunächst glatt und leicht reinigbar zu halten.

Beim Ankoppeln der externen Funktionseinrichtung an die Abdichtungseinrichtung jedoch kann der Bolzen des Aufnahmeabschnitts lokal gegen die Abdichtungseinrichtung drücken, diese lokal in Richtung zur externen Funktionseinrichtung ausbeulen und somit den Ventileinsatz eindrücken, unter Umständen bis zum Anschlag in das Hartteil der externen Funktionseinrichtung.

Die Abdichtungseinrichtung kann während des angekoppelten Zustands der externen Funktionseinrichtung in dem lokal ausgebeulten oder vorstehenden Zustand verbleiben.

Eine Befestigung des Bolzens durch Verschrauben, z.B. mittels eines Feingewindes, kann vorteilhaft eine definierte Einstellung des gewünschten Verschiebungsweges des Ventileinsatzes erlauben.

Der Bolzen kann eine verbreiterte Ventilabstützung aufweisen, deren Durchmesser größer ist als das Feingewinde oder der Einpresszapfen.

In einer weiter bevorzugten Ausführungsform ist die Abdichtungseinrichtung zum funktionellen Ankoppeln wenigstens einer externen Funktionseinrichtung ausgestaltet.

Der Begriff "Ankoppeln", wie er hierin verwendet wird, kann ein funktionelles und/oder mechanisches Verbinden der externen Funktionseinrichtung mit einem Koppelpartner auf der Seite der Anordnung mit einer Wechselwirkung und/oder Signalkommunikation und/oder Fluidverbindung zwischen externer Funktionseinrichtung und Anordnung im angekoppelten Zustand der externen Funktionseinrichtung bezeichnen oder umfassen.

Ein "Koppelpartner der Anordnung" kann zum Beispiel eine Messeinrichtung, wie beispielsweise ein Sensor, sein. Es kann eine Fluidleitung und dergleichen mehr sein.

Die externe Funktionseinrichtung kann ein Kassettensystem sein. Sie kann zur Verwendung in einem Hämodialyseverfahren geeignet sein. Ein solches Kassettensystem kann aus einem soliden, dreidimensionalen Kassettenkörper aus Kunststoff oder einem anderen Hartmaterial bestehen. Es kann zu einer Seite hin offen sein und zahlreiche Konnektionsöffnungen zum Einstecken, Einkleben und/oder Einschweißen von Schläuchen aufweisen.

Auf der offenen Seite eines solchen Kassettenkörpers kann eine Folie, wie beispielsweise eine Kunststofffolie, aufgebracht werden. Die Folie kann beispielsweise aufgeklebt und/oder aufgeschweißt werden. Unter Verwenden einer solchen Folie können durch den Kassettenkörper und die Folie Kanäle und Kammern gebildet werden, durch welche Fluide, wie beispielsweise Blut und/oder Substituatflüssigkeit, geleitet werden können.

Die Folie kann nur an einem äußeren Rand an dem Kassettenkörper befestigt sein und/oder an allen Kanalbegrenzungen. Es kann gewünscht und vorteilhaft sein, die Folie an bestimmten Stellen nicht zu befestigen, welche für im Betrieb der Anordnung zu öffnende Bereiche zum Ausführen und/oder Gewährleisten einer Ventilfunktion vorgesehen sind.

An Konnektionsöffnungen des Kassettenkörpers können Schläuche, wie beispielsweise Kunststoffschläuche, befestigt werden, so dass den mit den Öffnungen verbundenen Kanälen Fluide zu- und/oder aus diesen abgeleitet werden können.

Aufgrund des Aufbaus der Kassette mit solidem Kassettenkörper auf der einen und einer flexiblen Folie aus der anderen Seite kann es möglich sein, diverse Sensoren zur Erfassung verschiedener Messgrößen in den Kanälen und Kammern und/oder Aktoren zur Beeinflussung der Flüssigkeitsströme der Kanäle an die Folie funktionell anzukoppeln.

Ein funktionelles Ankoppeln der externen Funktionseinrichtung an der Anordnung kann in geeigneter Weise durch mechanisches Ankoppeln, wie beispielsweise durch Verpressen der AS-Platte der Anordnung mit der externen Funktionseinrichtung, erreicht werden.

Dies kann insbesondere bedeuten, dass die Folie der externen Funktionseinrichtung mit der Abdichtungseinrichtung in körperlichen Kontakt gebracht wird und auf dieser aufliegt.

Da es durch den direkten Kontakt der Materialien der Abdichtungseinrichtung und der Folie zu Messungenauigkeiten kommen kann, sind die Materialien in einer bevorzugten Ausführungsform vorzugsweise ausgewählt, um den Reibungskoeffizienten in vorteilhafter Weise zu reduzieren. Dies kann beispielsweise durch eine nachträgliche Beschichtung oder Oberflächenmodifizierung der Abdichtungseinrichtung erreicht werden. Auf diese Weise kann eine Weiterleitung von Scherspannung auf z. B. einen Drucksensor vermieden werden.

Daneben kann das Verpressen bzw. Einspannen der externen Funktionseinrichtung zur Folie hin notwendig sein, um die Dichtigkeit der Kanäle der externen Funktionseinrichtung zumindest in Teilbereichen sicherzustellen. Dies kann durch Anpressen bzw. Verpressen der Folie an die externe Funktionseinrichtung erreicht werden. Ferner kann eine Abstützung des fluidischen Innendrucks der externen Funktionseinrichtung erreicht werden.

Zum funktionellen Ankoppeln der externen Funktionseinrichtung an der Anordnung kann diese in geeigneter Weise zunächst auf der AS-Platte positioniert und gegebenenfalls fixiert und anschließend mit dieser verpresst werden. Auf diese Weise können die in und/oder an der AS-Platte vorgesehenen und/oder montierten Sensoren und/oder Aktoren und/oder Teile derselben an die externe Funktionseinrichtung und insbesondere an die Folie derselben ankoppeln. Als Ankoppelpartner kann hierbei wie auch bei anderen Ausführungsformen eine elastische Abdichtungseinrichtung, wie beispielsweise eine elastische Matte aus Silikon, dienen.

Ebenso wie die Ankopplung der Abdichtungseinrichtung an die AS-Platte und/oder Komponenten derselben kann auch die Abdichtungseinrichtung mittels Anlegen eines Unterdrucks an die Folie der externen Funktionseinrichtung angekoppelt werden. Siehe dazu auch die Patentanmeldung DE 10 2007 042 964 der Anmelderin auch der vorliegenden Erfindung.

Die luftfreie Ankopplung der Abdichtungseinrichtung an die Folie der externen Funktionseinrichtung kann jedoch auch ohne aktives Absaugen des Zwischenraums zwischen der Abdichtungseinrichtung und der Folie erfolgen. Beispielsweise können die externe Funktionseinrichtung und die Abdichtungseinrichtung aufgrund ihrer geometrischen Ausformung luftfrei aneinander gedrückt werden. Sie können auf diese Weise geeignet miteinander verpresst werden.

Eine korrekte Ankopplung und sogar eine Leckage von Luft bzw. Flüssigkeiten, wie beispielsweise Blut und/oder Substituat, in dem Zwischenraum zwischen der Abdichtungseinrichtung und der Folie der externen Funktionseinrichtung können mit Hilfe eines Sensors erfasst werden. Siehe dazu auch die Patenanmeldung DE 10 2008 062 037, ebenfalls von der Anmelderin der vorliegenden Erfindung.

Die Abdichtungseinrichtung kann in geeigneter Weise ausgelegt und vorgesehen sein, um aufgrund ihrer Elastizität als Membran zu fungieren. Sie kann sich in geeigneter Weise verformen und/oder aufgrund der Druckdifferenz zwischen der Oberfläche und der Unterseite der Membran bzw. der Abdichtungseinrichtung Kräfte zu einem Sensor leiten. Ein solcher Sensor kann an der externen Funktionseinrichtung und/oder an der AS-Platte vorgesehen sein.

Die Sensoren und/oder Aktoren der Abdichtungseinrichtung können in geeigneter Weise dazu ausgelegt und vorgesehen sein, um mit der AS-Platte verbunden zu werden.

In einer weiter bevorzugten Ausführungsform der vorliegenden Erfindung ist vorgesehen, dass die Abdichtungseinrichtung wenigstens einen Sensor und/oder Aktor und/oder eine Leitung und/oder wenigstens einen Teil wenigstens eines derselben aufweist.

"Sensoren", wie sie hier bezeichnet sind, schließen, wiederum ohne darauf beschränkt zu sein, elektrische, optische, akustische, mechanische, chemische, virtuelle und/oder digitale Sensoren, Messwertaufnehmer und/oder Messwertfühler und dergleichen ein.

Als mechanischer Sensor kann beispielsweise ein Schalter eingesetzt werden. Chemische Sensoren können beispielsweise dann eingesetzt werden, wenn eine Matte oder Folie für Gasdiffusion durchlässig ist und eine derartige Diffusion eines Gases gemessen werden soll.

Unter dem Begriff "virtueller Sensor" kann ein mittels Software realisierter Sensor oder eine erzielte Wirkung eines solchen verstanden werden, welcher Werte "misst", die aus den Messwerten realer Sensoren mit Hilfe eines empirisch erlernten oder physikalischen Modells abgeleitet werden. Virtuelle Sensoren eignen sich bevorzugt für Anwendungen, in denen reale Sensoren zu teuer sind, oder in Umgebungen, in denen reale Sensoren nicht bestehen können oder vorzeitig verschleißen.

Ein Sensor kann bestimmte physikalische und/oder chemische Eigenschaften und/oder eine Änderung derselben, wie eine Zunahme oder eine Abnahme eines Effektes, einer Ausprägung, einer Beschaffenheit und dergleichen, wie beispielsweise Temperatur, Druck, Feuchtigkeit, optische Signale, wie beispielsweise Helligkeit und/oder eine optische Änderung einer Zusammensetzung, Wärmestrahlung, Schall, Durchflussmengen, und/oder die stoffliche Beschaffenheit seiner Umgebung qualitativ oder quantitativ erfassen.

Sensoren im Sinne der vorliegenden Erfindung können als passive Sensoren oder aktive Sensoren ausgelegt sein.

Sensoren im Sinne der vorliegenden Erfindung können beispielsweise Drucksensoren sein, welche auch Drücke unterhalb des Umgebungsdrucks aufnehmen.

Sensoren im Sinne der vorliegenden Erfindung können Drucksensoren, Füllstandsensoren, optische Sensoren, Ultraschallsensoren, Temperatursensoren und dergleichen mehr sein.

Sensoren im Sinne der vorliegenden Erfindung können kapazitive Sensoren sein, die den Fluidpegel, insbesondere den Flüssigkeitspegel, in den aus dem Hartteil der externen Funktionseinrichtung und der Folie der externen Funktionseinrichtung gebildeten Kammern erfassen. Geeignete kapazitive Sensoren können durch die Abdichtungseinrichtung hindurch Dielektrizitätsunterschiede detektieren.

Die Abdichtungseinrichtung weist in einer bevorzugten erfindungsgemäßen Ausführungsform wenigstens eine Abdünnung auf. Die Abdünnung kann zur Unterstützung von Rückschlagventilen ausgestaltet sein. Sie kann eine geringere Nachgiebigkeit als andere Bereiche aufweisen. Sie kann beispielsweise 0,5 bis 1 mm tief sein. Die Abdünnung kann über einem Drucksensor vorgesehen sein.

Die Abdünnung kann zur Reduzierung der Verpresseinflüsse auf die Druckmessung dienen.

Die Abdichtungseinrichtung kann wenigstens eine Vorformung für Ventile oder Kammerleitungen aufweisen.

Die Abdichtungseinrichtung kann wenigstens eine Aussparung aufweisen. Die Aussparung kann für eine permanente Kanalöffnung ausgestaltet sein. Sie kann 1 mm tief sein.

Die Abdichtungseinrichtung weist vorzugsweise eine Vorformung für ein Post-Dilutions-Ventil auf. Sie weist vorzugsweise eine Vorformung für ein SN-Ventil (Single-Needle-Ventil). Sie weist vorzugsweise eine Vorformung für ein Prä-Dilutions-Ventil auf.

Die Abdichtungseinrichtung weist vorzugsweise eine Befestigungseinrichtung zum Befestigen einer externen Funktionseinrichtung hieran auf. Die Befestigungseinrichtung kann ein Positionierpin sein.

Die Abdichtungseinrichtung weist vorzugsweise einen Substituatkonnektor auf. Dieser kann ein automatischer Substituatkonnektor sein.

Die Abdichtungseinrichtung weist vorzugsweise eine Freistellung zum Einleiten eines Vakuums und/oder zum Evakuieren der Folie der Disposable-Kassette auf.

Der Aufnahmeabschnitt kann Teil einer Blutbehandlungsvorrichtung sein.

Die vierte Verbindungseinrichtung des Aufnahmeabschnitts kann eine Kerbe zum Aufnehmen der als Dichtlippe ausgestalteten dritten Verbindungseinrichtung sein. Die Dichtlippe der Abdichtungseinrichtung und die Kerbe des Aufnahmeabschnitts können zusammen eine Vakuumdichtung bilden.

Der Aufnahmeabschnitt weist vorzugsweise Unterstützungen für Rückschlagventile auf.

Der Aufnahmeabschnitt und oder die Abdichtungseinrichtung können verschiedene Ventile aufweisen, z.B. ein Post-Dilutions-Ventil, ein SN-Ventil und/oder ein Prä-Dilutions-Ventil. Das Post-Dilutions-Ventil und das Prä-Dilutions-Ventil können jeweils einen Durchmesser von 5 mm bis 25 mm, vorzugsweise 9 mm, aufweisen. Das SN-Ventil kann einen Durchmesser von 5 mm bis 40 mm, vorzugsweise 18 mm, aufweisen.

Der Aufnahmeabschnitt und/oder die Abdichtungseinrichtung können verschiedene Sensoren aufweisen. Hierzu zählen beispielhaft ein SN-Drucksensor, ein SN-Leveldetektor, ein Leveldetektor, beispielsweise zum Erfassen des Blutlevels einer venösen Kammer, Sensoren zum Erfassen der angekoppelten externen Funktionseinrichtung, ein venöser Drucksensor, ein arterieller Drucksensor, ein Ankoppelsensor und/oder ein Prä-Filter-Drucksenor.

Der SN-Drucksensor kann vorzugsweise sowohl einen Überdruck als auch einen Unterdruck erfassen, vorzugsweise von -333 mbar bis +1000 mbar, besonders bevorzugt von -266 mbar bis +800 mbar.

Der venöse Drucksensor kann vorzugsweise sowohl einen Überdruck als auch einen Unterdruck von -333 mbar bis +1000 mbar erfassen. Der venöse Drucksensor kann vorzugsweise sowohl einen Überdruck als auch einen Unterdruck erfassen, vorzugsweise von -266 mbar bis +800 mbar.

Der arterielle Drucksensor kann vorzugsweise sowohl einen Überdruck als auch einen Unterdruck von -733 mbar bis +600 mbar erfassen, besonders bevorzugt von -533 mbar bis +533 mbar.

Der Prä-Filter-Drucksensor kann vorzugsweise einen Druck (Unter- sowie Überdruck) von -133 mbar bis +2868 mbar erfassen. Der Prä-Filter-Drucksensor kann vorzugsweise einen Druck bis +1500 mbar erfassen.

Der Aufnahmeabschnitt weist vorzugsweise eine Absaug-Öffnung zum Absaugen von Luft zwischen dem Aufnahmeabschnitt und der Abdichtungseinrichtung auf. Die Absaug-Öffnung kann zum Erzeugen eines Unterdrucks von -680 mbar bis -700 mbar geeignet sein. Die Absaug-Öffnung kann zum Erzeugen eines Unterdrucks geeignet sein, der mindestens dem kleinsten zu messenden Unterdruck entspricht, vorzugsweise von -500 mbar bis -800 mbar.

Die Absaug-Öffnung kann zum Erzeugen eines Unterdrucks von vorzugsweise -500 mbar bis -800 mbar geeignet sein.

Die Abdichtungseinrichtung weist vorzugsweise einen Stempel für den Ankoppelsensor auf. Der Ankoppelsensor kann ein optischer Sensor sein. Der Stempel kann beweglich sein. Der Stempel kann durch Druckkraft bewegt werden.

Die Abdichtungseinrichtung weist vorzugsweise einen Stempel für den Sensor zum Erfassen bzw. Erkennen der angekoppelten externen Funktionseinrichtung auf. Der Stempel kann über Anpressung beweglich sein.

Der Sensor zum Erfassen der angekoppelten externen Funktionseinrichtung kann baugleich mit dem Ankoppelsensor ausgestaltet sein.

Die Abdichtungseinrichtung kann einen Niederhalter für den Drucksensor aufweisen. Die Abdünnung über dem Drucksensor kann vorzugsweise 0,1 mm bis 1,5 mm, besonders bevorzugt 0,5 mm betragen. Diese Werte können eine relative Abdünnung im Sinne einer Verdünnung gegenüber einem nicht abgedünnten Bereich bezeichnen. Sie können alternativ als absolute Werte, also als geometrische Angaben für die Dicke oder Stärke der Abdünnung zu verstehen sein.

Die externe Funktionseinrichtung weist vorzugsweise eine Kavität für ein Rückschlagventil auf. Das Rückschlagventil kann beispielsweise aus Silikon gebildet sein.

"Aktoren" im hier gebrauchten Sinn schließen, ohne darauf beschränkt zu sein, mechanische und/oder pneumatische und/oder elektrische Komponenten, wie beispielsweise Ventile, Stell- und/oder Regelglieder, Stellmotoren, Druckkolben, und dergleichen ein.

Leitungen, wie sie erfindungsgemäß eingesetzt werden können, können, ohne darauf beschränkt zu sein, Fluidleitungen, wie beispielsweise blutführende Kanäle, Substituatleitungen, elektrische Leitungen, Leitungen zur Signalkommunikation, wie beispielsweise Glasfaserkabel, und dergleichen umfassen.

In einer weiter bevorzugten Ausführungsform der vorliegenden Erfindung sind Sensoren und/oder Aktoren und/oder Leitungen und/oder Teile derselben form- und/oder kraft-und/oder stoffschlüssig mit der Abdichtungseinrichtung verbunden.

Eine geeignete Verbindung kann beispielsweise durch Integrieren der Sensoren und/oder Aktoren und/oder Leitungen und/oder Teile derselben direkt in die Abdichtungseinrichtung erreicht werden. Beispielsweise können Sensoren und/oder Teile derselben mit der Abdichtungseinrichtung vergossen und/oder in diese einvulkanisiert werden. Auf diese Weise kann in vorteilhafter Weise erreicht werden, dass die Messstrecke zwischen dem Sensor und der Abdichtungseinrichtung um die Ankoppelstelle des Sensors in der Abdichtungseinrichtung reduziert wird.

In einer weiter bevorzugten Ausführungsform der vorliegenden Erfindung weist die Abdichtungseinrichtung wenigstens eine Aufnahme für eine Befestigungseinrichtung der externen Funktionseinrichtung und/oder des Aufnahmeabschnitts auf.

Eine "Befestigungseinrichtung", wie sie hierin geeignet verwendet und vorgesehen ist, kann wenigstens ein Element sein, das ausgewählt ist aus einer Gruppe von Elementen wie Positionierungsstiften, Substituatkonnektoren, Positionierungsdornen, Schrauben, Spangen, Keilen und dergleichen mehr.

Eine "Aufnahme" für eine solche Befestigungseinrichtung kann eine in der Abdichtungseinrichtung vorgesehene Öffnung, eine Durchgangsöffnung, ein Durchbruch, ein Schlitz, ein Spalt, ein Loch oder Ähnliches sein.

Das Kombinieren der Befestigungseinrichtung mit der Aufnahme, insbesondere das Einführen der Befestigungseinrichtung in die Aufnahme und/oder gegebenenfalls Fixieren der Befestigungseinrichtung in der Aufnahe, kann zur Positionierung und/oder Fixierung der externen Funktionseinrichtung und/oder der AS-Platte dienen.

Die für zum Aufnehmen solcher Befestigungseinrichtungen vorgesehenen Bereiche sind dabei bevorzugt außerhalb der blutführenden Bereiche der externen Funktionseinrichtung angeordnet.

In einer weiter bevorzugten Ausführungsform ist die Abdichtungseinrichtung als Mehrkomponenten-Abdichtungseinrichtung ausgestaltet oder weist ein solches Material auf.

Weiter bevorzugt kann sie wenigstens ein elastomeres Material aufweisen oder hieraus bestehen. Ein geeignetes elastomeres Material kann beispielsweise, ohne darauf beschränkt zu sein, Gummi, Silikon, Kautschuk, Kunststoff, PVC und dergleichen mehr sein.

Ein geeignetes elastomeres Material kann ferner flüssigkristalline Elastomere und/oder thermoplastische Elastomere umfassen.

Eine solche Mehrkomponenten-Abdichtungseinrichtung kann beispielsweise erhalten werden, indem harte Teile, wie beispielsweise Teile aus Kunststoff, mit einem elastomeren Material umspritzt und/oder in ein Elastomerteil geschnappt oder verklebt werden. Auf diese Weise kann eine stabile Struktur der Abdichtungseinrichtung mit in sich stabilen Komponenten entstehen.

Alternativ kann die Abdichtungseinrichtung vollständig aus dem elastomeren Material gebildet sein, wodurch in vorteilhafter Weise eine leichte Herstellbarkeit und geringere Herstellungskosten realisiert werden können.

Die Abdichtungseinrichtung kann eine formfeste elastische bzw. biegsame bzw. flexible und/oder bewegliche oder bewegbare Abdichtungseinrichtung sein. Die Abdichtungseinrichtung kann beispielsweise als eine Matte, beispielsweise als eine Maschinenmatte, ausgestaltet sein.

Die Abdichtungseinrichtung kann erfindungsgemäß derart ausgestaltet sein, dass sie die Ausbreitung eines elektrischen Feldes nicht wesentlich beeinflusst. Sie kann elektrisch isolierend ausgestaltet sein. Sie kann ausreichend empfindlich ausgelegt sein, um beispielsweise auch einen niedrigen Druck in der Anordnung und/oder der externen Funktionseinrichtung aufzunehmen und/oder zu vermitteln.

Die Abdichtungseinrichtung kann eine Dicke von 1,0 mm bis 10,0 mm vorzugsweise von 2,6 mm, aufweisen.

In einer weiter bevorzugten Ausführungsform kann die Abdichtungseinrichtung in den Verpressbereichen dicker (z.B. mit einer Dicke von 2,6 mm) ausgestaltet sein, in den Bereichen von Kammern, Leveldetektoren, Kanalaußenbereichen, Rückschlagventilen und dergleichen jedoch dünner (z.B. 0,1 mm bis 1,5 mm dick, vorzugsweise 1,0 mm dick) ausgestaltet sein. In Bereichen von Drucksensoren kann die Abdichtungseinrichtung z.B. 0,5 mm dick sein. Sie kann eine Shorehärte von 30° bis 80°, vorzugsweise von etwa 50° aufweisen.

In einer weiteren Ausführungsform muss eine Verpresskraft zum Verpressen der externen Funktionseinrichtung an dem Aufnahmeabschnitt in den Mittenachsen des Aufnahmeabschnitts von 0,5 kN bis 10 kN, vorzugsweise von etwa 5 kN, eingesetzt werden. Substituatventile bringen eine notwendige Kraft von etwa 5 N bis 50 N, vorzugsweise etwa 40 N, auf. Die Kraft des SN-Ventils beträgt 20 N bis 200 N, vorzugsweise 100 N.

In einer weiteren Ausführungsform ist eine erfindungsgemäß mögliche Kanalabdichtung der Abdichtungseinrichtung derart modifiziert, dass die Verpressung nur noch an den Ventilen gefordert ist. Auf diese Weise kann die Verpresskraft deutlich reduziert werden (bevorzugt unter etwa 2,5 kN). Dies ermöglicht vorteilhaft die Optimierungsmöglichkeiten im Bereich von Abdichtungseinrichtung, z.B. bezüglich Dicke, Geometrie und Materialwahl. Es ist somit vorteilhaft auch möglich, das Zusammenspiel von Abdichtungseinrichtung und Aufnahmeabschnitt weiter zu optimieren.

In einer bevorzugten Ausführungsform ist die Anordnung als eine Anordnung zum Behandeln medizinischer Fluide ausgestaltet. Insbesondere kann die erfindungsgemäße Anordnung als eine Dialysevorrichtung, eine Hämodialysevorrichtung, eine Vorrichtung zur Hämofiltration oder zur Hämodiafiltration und dergleichen ausgestaltet sein.

Die erfindungsgemäße Anordnung kann als Anordnung in der Labortechnik, wie beispielsweise eine Analysevorrichtung, wie eine Chromatographievorrichtung, eine Waage und dergleichen, und/oder als Anordnung in der Nahrungs- und/oder Arzneimittelherstellung ausgestaltet sein.

In einer bevorzugten Ausführungsform weist die erfindungsgemäße Anordnung eine Unterdruckeinrichtung zum Fixieren der Abdichtungseinrichtung an der Anordnung mittels Unterdruck auf.

Die Abdichtungseinrichtung ist in vorteilhafter Weise zum Abdichten wenigstens eines ersten Volumens einer Anordnung zur Behandlung wenigstens eines medizinischen Fluids gegen wenigstens ein zweites Volumen der Anordnung geeignet. Sie kann in vorteilhafter Weise eine Abdichtung der in einer externen Funktionseinrichtung vorgesehenen Kanäle zum Leiten von Fluiden bereitstellen.

Die Abdichtungseinrichtung kann in vorteilhafter Weise eine Vakuumabdichtung bereitstellen. Die Abdichtungseinrichtung kann in vorteilhafter Weise die Folie einer externen Funktionseinrichtung und/oder deren Evakuierung unterstützen.

Da die Abdichtungseinrichtung aus einem elastischen, vorzugsweise elastomeren, Material gebildet sein kann und/oder ein solches umfassen kann, kann sie in vorteilhafter Weise in wenigstens einem Abschnitt selbst als Sensor fungieren oder einen Teil eines Sensors darstellen. Dazu kann sie beispielsweise eine Druckweiterleitung und/oder Druckvermittlung bewirken. Aufgrund ihrer Materialbeschaffenheit kann sie jedoch auch eine Druckisolierung erwirken. Durch eine mechanische Verformung der Abdichtungseinrichtung kann diese in vorteilhafter Weise eine Druckvergleichsmessung, zum Beispiel unter Anbindung eines Ankoppelsensors, zulassen.

Die Abdichtungseinrichtung kann in vorteilhafter Weise ebenso eine Weiterleitung und/oder Isolierung von Temperaturen, elektrischen Spannungen und Strömen zulassen.

Ferner kann die Abdichtungseinrichtung, insbesondere wenn sie ein elastomeres Material aufweist, eine Kippbewegung des Aufnahmeabschnitts zum

Aufnehmen der externen Funktionseinrichtung zur Anordnung zulassen. Sie kann aufgrund ihrer Materialeigenschaften ferner einen Toleranzausgleich zwischen den verschiedenen Komponenten der Anordnung und/oder der externen Funktionseinrichtung, insbesondere eines Hartteils der externen Funktionseinrichtung, zulassen.

In vorteilhafter Weise kann es aufgrund der Flexibilität und/oder Bewegbarkeit oder Beweglichkeit einer elastomeren Abdichtungseinrichtung auch möglich sein, Bautoleranzen und Verbindungsübergänge im Bereich von Ventilen auszugleichen.

Da die Abdichtungseinrichtung elastische Eigenschaften aufweisen kann und über die AS-Platte hinausragen und mit angrenzenden Gehäuseteilen, zum Beispiel der Trägereinrichtung, wie einem Maschinen- oder Trägerrahmen verbunden sein kann, kann in vorteilhafter Weise eine glatte und leicht reinigbare Maschinenoberfläche realisiert werden.

Daneben können - weiter begünstigt durch das Herstellverfahren der Abdichtungseinrichtung oder ihre Abmessungen - in vorteilhafter Weise schwer zu reinigenden Hinterschnitte auftreten vermieden werden.

Ferner kann es in vorteilhafter Weise möglich sein, gegebenenfalls auch große Spalte und/oder Öffnungen, welche möglicherweise zur Realisierung von zueinander beweglichen Bauteilen erforderlich sind, zuverlässig und abgedichtet zu überbrücken. Dabei kann eine Hemmung oder Behinderung deren Beweglichkeit oder Bewegbarkeit vermieden werden. Dies kann insbesondere in vorteilhafter Weise durch die in der ersten Verbindungseinrichtung vorgesehenen Bereiche mit geringerer Dicke der Abdichtungseinrichtung gefördert werden.

Insbesondere wenn die Abdichtungseinrichtung geschlossen - also ohne Durchgangsöffnungen oder freibleibende Bereichen in einem inneren Bereich davon - ausgeführt ist, können in vorteilhafter Weise alle unter der Abdichtungseinrichtung angeordneten Komponenten weitestgehend vor mechanischen, elektrischen, chemischen und/oder Strahlungseinflüssen geschützt werden.

Wenn die Abdichtungseinrichtung als eine in einem Inneren hiervor offene Abdichtungseinrichtung ausgeführt ist, können in vorteilhafter Weise ausgewählte Sensoren direkt an die Folie der externen Funktionseinrichtung ankoppeln. Da die Abdichtungseinrichtung gut an beispielsweise der AS-Platte befestigbar ist und die offenen Bereich gegenüber durch die Abdichtungseinrichtung bedeckte Bereiche der AS-Platte abdichten kann, kann die Oberfläche der AS-Platte auch bei einer offenen Abdichtungseinrichtung gut reinigbar sein.

Die Abdichtungseinrichtung weist eine hohe Funktionsintegrierbarkeit auf. Insbesondere können für die Funktion einer Blutbehandlungsvorrichtung, wie einer Dialysemaschine, im Wechselspiel mit der externen Funktionseinrichtung wie einer Disposable-Kassette erforderlichen Funktionen in vorteilhafter Weise in die Abdichtungseinrichtung integriert werden.

Die durch die Abdichtungseinrichtung und die Folie der externen Funktionseinrichtung und die durch den direkten Kontakt der Materialien hervorgerufenen Beeinträchtigungen der Messgenauigkeit können bei der vorliegenden Abdichtungseinrichtung in vorteilhafter Weise durch verschiedene Maßnahmen reduziert werden. So kann es zum Beispiel in vorteilhafter Weise möglich sein, die Dicke der Abdichtungseinrichtung im Bereich der Sensoren deutlich zu verringern und/oder die Reibungskoeffizienten der Materialpartner (beispielsweise: Abdichtungseinrichtung und externe Funktionseinrichtung) herabzusetzen.

Daneben kann die Abdichtungseinrichtung insbesondere bei geometrie- und/oder materialbedingter konstanter mechanischer Rückstellkraft mittels Ankoppelsensor in vorteilhafter Weise das Vorhandensein einer externen Funktionseinrichtung erfassen. Derartige Ankoppelsensoren können auch im Aufnahmeabschnitt vorgesehen sein.

Eine vorzugsweise vorgesehene IR-Intransparenz der Abdichteinrichtung kann ebenfalls zum Erkennen einer externen Funktionseinrichtung beitragen.

Wenn ein Druck innerhalb der Kanäle und/oder Kammern der externen Funktionseinrichtung erfasst werden soll, können aufgrund der Druckleitungseigenschaften einer beispielsweise als Elastomer ausgestalteten und vorgesehenen Abdichtungseinrichtung die Sensoren in vorteilhafter Weise auch geschützt innerhalb der Abdichtungseinrichtung in der AS-Platte angeordnet und/oder fixiert werden. Die Abdichtungseinrichtung kann somit in vorteilhafter Weise eine Schutzfunktion für Sensoren und/oder Aktoren und/oder Leitungen vor äußeren Einflüssen darstellen. Dies kann einen Schutz für die Sensoren bewirken und darüber hinaus zu erhöhter Genauigkeit der mit den jeweiligen Sensoren gemessenen Werten führen.

Es kann besonders vorteilhaft sein, dass die Abdichtungseinrichtung eine sichere Abdichtung des Zwischenraums zwischen der Folie der externen Funktionseinrichtung und der Abdichtungseinrichtung gegen Leckageluft von Außen sicherstellen kann. Dies kann durch Vorsehen einer Dichtlippe an der Abdichtungseinrichtung bewirkt werden.

Wenn die Abdichtungseinrichtung mittels Unterdruck mit einer Auflagefläche wie der AS-Platte verbunden wird, kann die Abdichtungseinrichtung nach Aufheben des Unterdrucks in vorteilhafter Weise leicht von der AS-Platte und/oder anderen Komponenten gelöst werden. Auf diese Weise kann in vorteilhafter Weise ein Wechsel der Abdichtungseinrichtung aufgrund von Beschädigung und/oder Alterung oder ihre Entnahme zu Reinigungszwecken auf einfache Weise erfolgen.

Der erfindungsgemäß realisierbare Originalitätsverschluss kann in vorteilhafter Weise eine unsachgemäße Montage bzw. Demontage der Abdichtungseinrichtung verhindern. Zudem kann er in vorteilhafter Weise eine Keimverschleppung verhindern. Daneben kann eine gut zu reinigende Oberfläche erhalten werden. Da der Spalt bzw. die Öffnung in dieser Ausführungsform für den Anwender nicht zugänglich ist, kann in vorteilhafter Weise das Eindringen unerwünschter Partikel, wie Staub, und/oder von Fluiden, wie Substituatflüssigkeit und/oder Blut, besonders zuverlässig vermieden oder gar verhindert werden.

Wenn die Abdichtungseinrichtung als Mehrkomponenten-Abdichtungseinrichtung ausgeführt ist, können bestimmte Abschnitte der Absichtungseinrichtung in vorteilhafter Weise zur Positionierung, Befestigung, Verformung und unter Gesichtspunkten einer erhöhten Haltbarkeit gezielt und unabhängig voneinander ausgestaltet werden.

Im Folgenden werden bevorzugte Ausführungsformen der erfindungsgemäßen medizinische Anordnung und ihrer Abdichtungseinrichtung unter Bezugnahme auf die Figuren beschrieben. In der Zeichnung bezeichnen gleiche Bezugszeichen gleiche oder identische Elemente. Es gilt:
- Fig. 1: zeigt einen Teil-Querschnitt in einem seitlichen Bereich einer Abdichtungseinrichtung gemäß einer ersten Ausführungsform, die mit einer Anordnung verbunden ist;
- Fig. 2: zeigt einen Teil-Querschnitt in einem seitlichen Bereich einer Abdichtungseinrichtung gemäß einer zweiten Ausführungsform , die mit einer Anordnung verbunden ist;
- Fig. 3: zeigt einen Teil-Querschnitt in einem seitlichen Bereich einer Abdichtungseinrichtung gemäß einer dritten Ausführungsform , die mit der Anordnung und einer externen Funktionseinrichtung verbunden ist;
- Fig. 4: zeigt einen Teil-Querschnitt in einem seitlichen Bereich einer Abdichtungseinrichtung gemäß einer vierten Ausführungsform , die mit der Anordnung und einer externen Funktionseinrichtung verbunden ist;
- Fig. 5: zeigt eine Schnappverriegelung zur Verwendung mit der erfindungsgemäßen Anordnung mit Abdichtungseinrichtung;
- Fig. 6: zeigt schematisch eine Einrichtung zur Positionierung einer externen Funktionseinrichtung in einer Draufsicht auf die Abdichtungseinrichtung;
- Fig. 7: zeigt schematisch eine Verschnappung der Abdichtungseinrichtung bei nicht verschnappter Abdichtungseinrichtung in einem Querschnitt;
- Fig. 8: zeigt schematisch eine Verschnappung der Abdichtungseinrichtung bei verschnappter Abdichtungseinrichtung in einem Querschnitt;
- Fig. 9: zeigt eine Abdichtungseinrichtung mit einem Sensor im Querschnitt;
- Fig. 10: zeigt schematisch die Verbindung zwischen einem Aufnahmeabschnitts zwischen der Abdichtungseinrichtung und der externen Funktionseinrichtung in einem horizontalen Schnitt;
- Fig. 11: zeigt einen horizontalen Schnitt durch einen Phantomventil-Aktor, welcher nicht die Erfindung darstellt;
- Fig. 12: zeigt einen horizontalen Schnitt durch einen Phantomventil-Aktor einer erfindungsgemäßen Ausführungsform; und
- Fig. 13: zeigt schematisch vereinfacht einen Schnitt einer Ventileinrichtung, welche an eine erfindungsgemäße Anordnung mit Abdichtungseinrichtung gekoppelt ist.

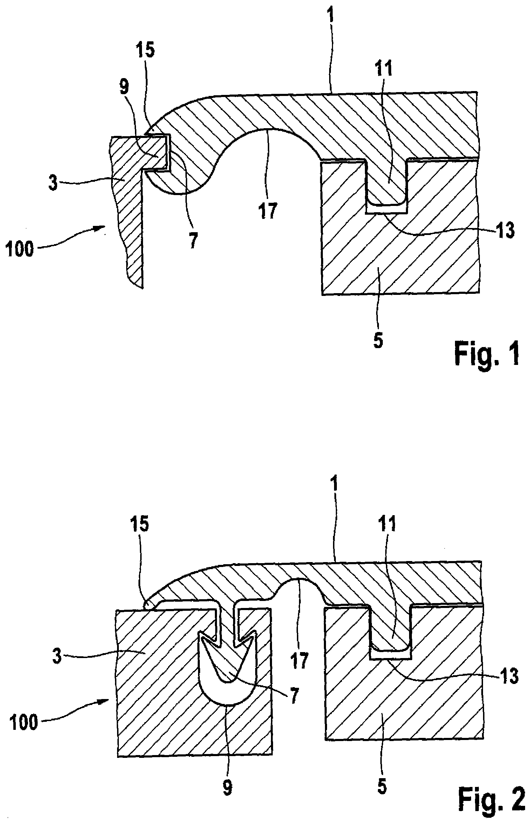

Fig. 1 zeigt im Querschnitt einen Abschnitt einer Abdichtungseinrichtung 1 gemäß einer ersten Ausführungsform hiervon, die mit einer Trägereinrichtung 3 einer Anordnung 100 und einem Aufnahmeabschnitt 5 der Anordnung 100 verbunden ist. Die Anordnung 100 kann eine Blutbehandlungsvorrichtung sein. Die Trägereinrichtung 3 der Anordnung 100 kann ein Trägerrahmen sein. Der Aufnahmeabschnitt 5 der Anordnung 100 kann eine AS-Platte sein.

Die Abdichtungseinrichtung 1 weist eine erste Verbindungseinrichtung 7 auf. Die erste Verbindungseinrichtung 7 ist eine Aufnahmeeinrichtung für eine einsteckbare Einrichtung. Die Trägereinrichtung 3 weist eine zweite Verbindungseinrichtung 9 auf. Die zweite Verbindungseinrichtung 9 ist eine einsteckbare Einrichtung.

Die Abdichtungseinrichtung 1 weist eine dritte Verbindungseinrichtung 11 auf. Die dritte Verbindungseinrichtung 11 ist wiederum eine einsteckbare Einrichtung. Der Aufnahmeabschnitt 5 weist eine vierte Verbindungseinrichtung 13 auf. Die vierte Verbindungseinrichtung 13 ist eine Aufnahmeeinrichtung für die als einsteckbare Einrichtung ausgestaltete dritte Verbindungseinrichtung 11 der Abdichtungseinrichtung 1.

Die Abdichtungseinrichtung 1 weist eine Überhanglippe 15 zum Übergang zur Trägereinrichtung 3 auf.

Die Abdichtungseinrichtung 1 weist im Bereich der ersten Verbindungsvorrichtung 7 einen Bereich 17 mit geringerer Dicke - gemessen an anderen Abschnitten der Abdichtungseinrichtung 1 - auf.

Fig. 2 zeigt im Querschnitt einen Abschnitt einer Abdichtungseinrichtung 1 gemäß einer zweiten Ausführungsform hiervon.

Die Abdichtungseinrichtung 1 der zweiten Ausführungsform entspricht im Wesentlichen derjenigen der ersten Ausführungsform. Sie unterscheidet sich von jener der Fig. 1 dadurch, dass die erste Verbindungseinrichtung 7 der Abdichtungseinrichtung 1 als einsteckbare Einrichtung ausgestaltet ist und die zweite Verbindungsvorrichtung 9 der Trägereinrichtung 3 als Aufnahmeeinrichtung für die als einsteckbare Einrichtung ausgestaltete erste Verbindungseinrichtung 7 ausgestaltet ist.

Die erste Verbindungseinrichtung 7 ist in der zweiten Ausführungsform der Figur 2 ein Widerhaken.

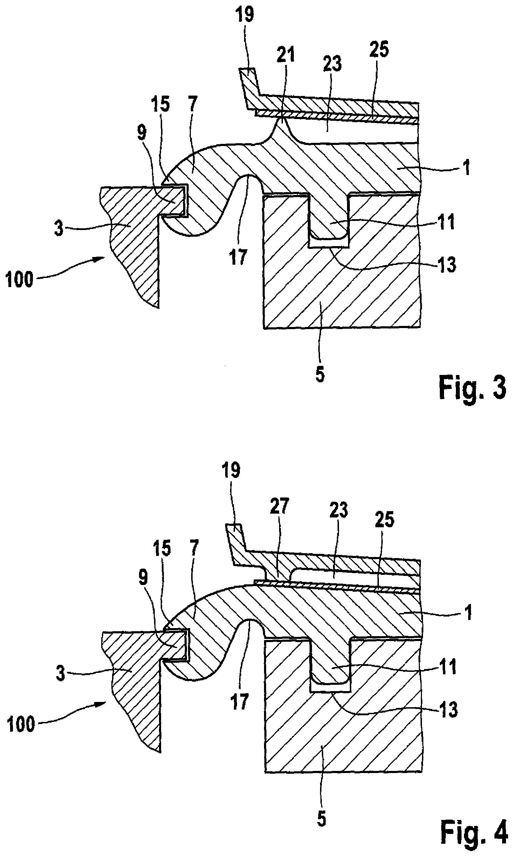

Fig. 3 zeigt einen Querschnitt in einem seitlichen Bereich einer Abdichtungseinrichtung 1 gemäß einer dritten Ausführungsform hiervon. Die Abdichtungseinrichtung 1 ist mit der Anordnung 100 und einer externen Funktionseinrichtung 19 verbunden. Die externe Funktionseinrichtung 19 kann eine blutführende Disposable-Kassette sein.

Die Abdichtungseinrichtung 1 weist eine Vakuumdichtung 21 zum Abdichten eines Zwischenraums 23 zwischen einer Folie 25 der externen Funktionseinrichtung 19 und der Abdichtungseinrichtung 1 auf. Die Vakuumdichtung 21 steht mit der Folie 25 in Kontakt. Die Vakuumdichtung 21 kann als Dichtlippe ausgestaltet sein.

Der Zwischenraum 23 kann evakuiert werden, um die externe Funktionseinrichtung 19 mit der Abdichtungseinrichtung 1 zu verbinden und die externe Funktionseinrichtung 19 funktionell an die Anordnung 100 anzukoppeln.

Fig. 4 zeigt einen Ausschnitt einer Abdichtungseinrichtung 1 gemäß einer vierten Ausführungsform hiervon im Querschnitt, die mit der Anordnung 100 und einer externen Funktionseinrichtung 19 verbunden ist.

Die Abdichtungseinrichtung 1 gemäß der vierten Ausführungsform entspricht im Wesentlichen derjenigen der dritten Ausführungsform. Sie unterscheidet sich dadurch, dass statt der Vakuumdichtung 21 an der Abdichtungseinrichtung 1 eine Vakuumdichtung 27 an der externen Funktionseinrichtung 19 vorgesehen ist, um den Zwischenraum 23 abzudichten. Die Vakuumdichtung 27 ist exemplarisch als Dichtwulst ausgestaltet.

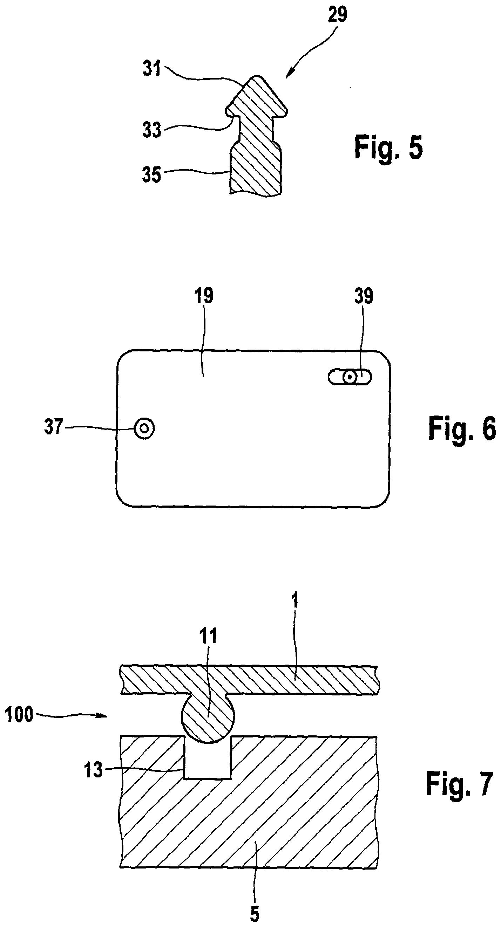

Fig. 5 zeigt eine Schnappeinrichtung 29 oder einen Teil hiervon in Gestalt eines Pins. Die Schnappeinrichtung 29 weist eine Einführschräge 31, einen Hinterschnitt 33 sowie einen Abschnitt 35 zum Ausrichten und/oder Positionieren der externen Funktionseinrichtung auf (nicht gezeigt). Mittels der Einführschräge 31 ist ein einfaches Einführen des Pins in eine Öffnung möglich, mittels des Hinterschnitts 33 kann der Pin in der Öffnung eingeschnappt und in dieser gehalten werden. Der Abschnitt 35 kann ein Ausrichten oder Positionieren der externen Funktionseinrichtung beim Verschnappen derselben sicherstellen.

Eine Schnappeinrichtung 29 wie beispielsweise der in Fig. 5 gezeigte Pin kann auch Teil der Abdichtungseinrichtung 1 sein und ihre Ausrichtung und/oder Verschnappung an der externen Funktionseinrichtung 19 oder an einem Abschnitt der Anordnung 100 bewirken. Eine Schnappeinrichtung 29 kann eine oben beschriebenen Originalitätsverschluss bilden oder ein Teil eines solchen sein.

Fig. 6 zeigt eine Fläche, z.B. eine Unterseite, einer Kassette als Beispiel einer externen Funktionseinrichtung 19. Auf der Fläche ist ein erster Positionierpin 37 als Fixpunkt oder Ursprung eines Koordinatensystems und ein zweiter Positionierpin 39 mit Langloch zum Toleranzausgleich vorgesehen.

Fig. 7 zeigt eine Verschnappungseinrichtung der Abdichtungseinrichtung 1 welche eine dritte Verbindungseinrichtung 11 aufweist, mit einem Aufnahmeabschnitt 5, welcher eine vierte Verbindungseinrichtung 13 aufweist. Die Verschnappungseinrichtung ist in Fig. 7 in einem nicht verschnappten bzw. verbundenen Zustand gezeigt.

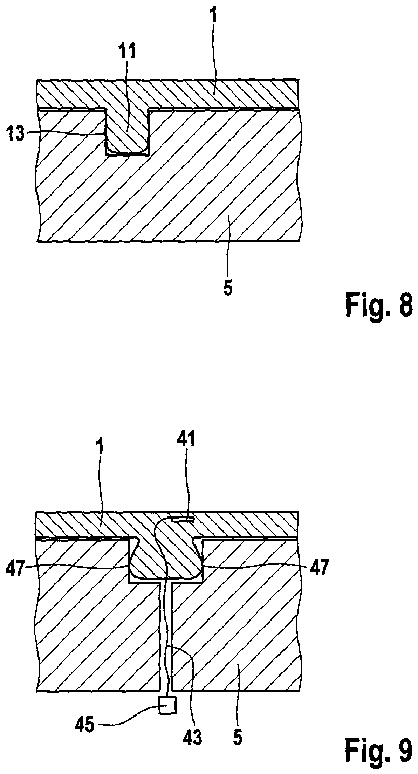

Fig. 8 zeigt die Verschnappungseinrichtung der Fig. 7 der Abdichtungseinrichtung 1 in einem verschnappten bzw. verbundenen Zustand. Gut zu erkennen beim Vergleich der Fig. 7 mit der Fig. 8 ist, dass die im Querschnitt vormals teilkreisförmige dritte Verbindungseinrichtung 11 (siehe Fig. 7) eine nach Verschnappung (siehe Fig. 8) veränderte Querschnittform aufweist. Die Formveränderung trägt zur Abdichtung bei. Sie kann vorzugsweise auch zu einer Befestigung der Abdichtungseinrichtung 1 am Aufnahmeabschnitt 5 beitragen.

Fig. 9 zeigt eine Abdichtungseinrichtung 1 mit einem Sensor 41 im Querschnitt. Der Sensor 41 ist, wie in Fig. 9 gezeigt, in ein Inneres der Abdichtungseinrichtung 1 eingebettet. Er kann als ein Temperatursensor ausgestaltet sein. Der Sensor 41 ist mittels einer Zuleitung 43 mit einem Äußeren der Abdichtungseinrichtung 1 verbunden. Er weist in Fig. 9 einen Steckverbinder 45 zum Herstellen einer Signalverbindung auf.

An den mit dem Bezugszeichen 47 gekennzeichneten Stellen ist mittels der Verschnappung, wie sie in den Fig. 7 und 8 gezeigt ist, eine Vakuumdichtung hergestellt.

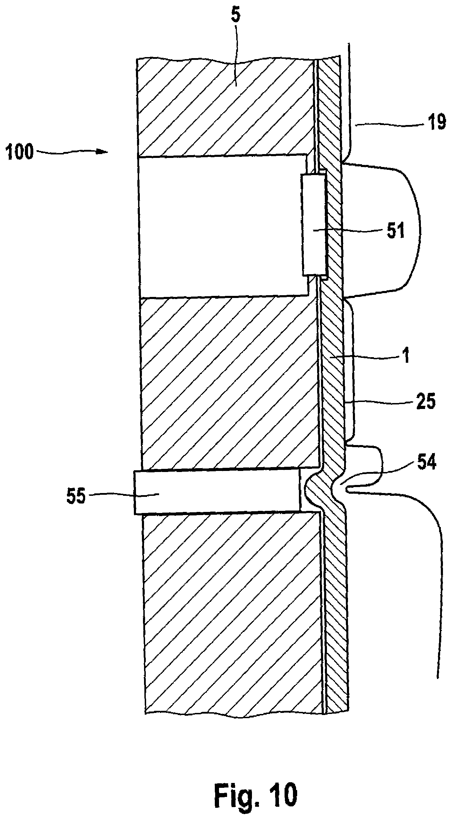

Fig. 10 zeigt schematisch den Aufbau eines Aufnahmeabschnitts 5, der Abdichtungseinrichtung 1 und der externen Funktionseinrichtung 19 in einem horizontalen Schnitt.

Der Aufnahmeabschnitt 5 weist einen Sensor 51 (hier ein Drucksensor) und einen Stößel 55 auf. Der Stößel 55 kann ein Ventilstößel sein.

Durch Bewegungen des Stößels 55 kann mittels eines Phantomventils 54 in der Abdichtungseinrichtung 1 ein Unterdruck zum Halten der Abdichtungseinrichtung 1 erzeugt werden.

Beispiele für Sensoren 51 des Aufnahmeabschnitts 5 schließen ungeachtet weiterer Merkmale der jeweiligen Ausführungsform und ohne darauf beschränkt zu sein als anordnungsseitige Sensoren, die durch die Abdichtungseinrichtung messen, z.B. einen kapazitativer Level-Detektor für die Single-Needle-Kammer der externen Funktionseinrichtung, Drucksensoren, Ultraschallsensoren, optische Sensoren und dergleichen ein.

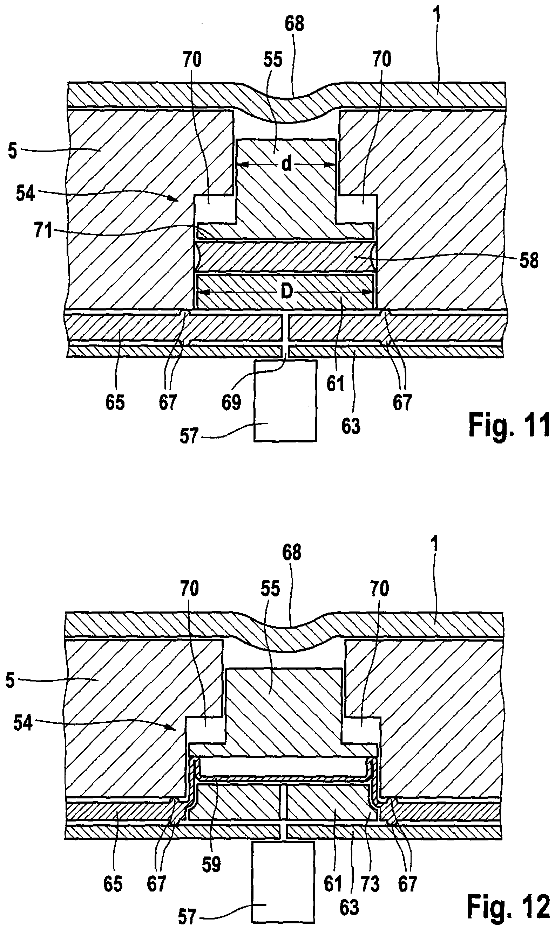

Die Figuren 11 bzw. 12 zeigen horizontale Schnitte durch einen Phantomventil-Aktor in einer ersten bzw. einer zweiten Ausführungsform desselben, wobei nur die zweite Ausführungsform erfindungsgemäß ist.

Der Stößel 55 kann sich mittels Kraftübertragung durch ein Ventil 57 bewegen. Das Ventil 57 kann ein Vorsteuerventil sein. Der Stößel 55 ist T-förmig ausgestaltet. Der Durchmesser d des Stößels 55 im längeren Abschnitt der T-Form kann beispielsweise 5 mm bis 40 mm, vorzugsweise 18 mm, (bei einem SN-Ventil) oder 5 mm bis 25 mm, vorzugsweise 9 mm, (bei einem Substituatventil) betragen.

Es ist ein scheibenförmiges Element 58 vorgesehen, das zwei ringförmige umlaufende Vorsprünge aufweist. Die beiden ringförmig umlaufenden Vorsprünge wirken in radialer Richtung gegen die Zylinderinnenwand. Erfindungsgemäß kann hierbei eine andere Anzahl von ringförmig umlaufenden Ringen bzw. ringförmige umlaufende Vorsprüngen vorgesehen sein, z.B. ein Ring, drei Ringe oder mehr.

Ferner ist ein Füllkörper 61 vorgesehen. Der Füllkörper 61 kann einen Durchmesser D von beispielsweise 10 mm bis 60 mm, vorzugsweise 25 mm, (SN-Ventil) oder von beispielsweise 10 mm bis 50 mm, vorzugsweise 16 mm, (Substituatventil) aufweisen. Zwischen dem Ventil 57 und dem Füllkörper 61 sind eine Luftverteilerplatte 63 und eine Dichtmatte 65 mit Dichtwulsten 67 vorgesehen.

Die Betätigung des Phantomventil-Aktors kann zu einer Verformung 68 der Abdichtungseinrichtung 1 führen.

Über eine Öffnung 69 kann das Ventil 57 Luft bzw. Gas ansaugen oder zuführen. Der Stößel 55 kann bei seiner Bewegung nach unten oder oben in der Fig. 11 die Abdichtungseinrichtung 1 nach unten ziehen oder nach oben drücken bzw. ihre Bewegung nach oben erlauben.

Da das scheibenförmige Element 58 wie oder als radial wirkende Dichtringe gegen die zylindrische Innenwand wirkt ist der Stößel 55 nicht frei in seiner Bewegung innerhalb der Bohrung oder Sacköffnung 70.

Durch das scheibenförmige Element 58 kann ein gas- oder luftdichter Raum 71 geschaffen werden, der das Erzeugen eines Unterdrucks zulässt.

Mit Hilfe der Luftverteilerplatte 63 wird nur an vorbestimmten Stellen ein Unterdruck zum Ansaugen der Abdichtungseinrichtung 1 oder ein Überdruck zum Abheben der Abdichtungseinrichtung 1 erzeugt.

Die Bohrung oder Sacköffnung 70 an ihrer zylindrischen Innenwand in geeigneter Weise oberflächenbehandelt, z.B. vorzugsweise rolliert oder poliert, um eine bevorzugte Oberflächenbeschaffenheit für das daran entlang gleitende scheibenförmige Element 58 für eine geeignete Dichtung zu erzielen.

Fig. 12 zeigt einen horizontalen Schnitt durch eine zweite Ausführungsform eines Phantomventil-Aktors; Fig. 12 zeigt eine Ausführungsform der Erfindung.

Es ist eine Aktor-Membran 59 vorgesehen, die als Dichteinrichtung fungieren kann. Anders als in der Ausführungsform der Fig. 11 ist die Dichtmatte 65 in Fig. 12 gemeinsam mit der integrierten Aktor-Membran 59 ausgestaltet. Der Füllkörper 61 weist ferner Anschläge 73 auf.

Die in die Dichtmatte 65 integrierte Aktor-Membran 59 ist dazu ausgelegt, sich unter Druck auszudehnen und den Stößel 55 im Bezugssystem der Fig. 12 nach oben zu drücken oder ihn oben halten. Bei Unterdruck kann sich die Aktor-Membran 59 zusammenziehen oder zusammenfallen und den Stößel 55 im Bezugssystem der Figur nach unten ziehen oder deren Bewegung in dieser Richtung zulassen. Auf diese Weise kann die Abdichtungseinrichtung 1 ausgewölbt, zumindest aber an- oder eingesaugt werden.

Der Stößel 55 kann sich in einer Anordnung gemäß Fig. 12 in der Sacköffnung 70 in deren axialer Richtung frei bewegen.

An die zylindrische Innenwand der Bohrung oder Sacköffnung 70 sind bei dieser Ausführungsform keine besonderen Anforderungen an die Oberflächenbeschaffenheit zu stellen, was das Herstellverfahren der Sacköffnung 70 vorteilhaft vereinfachen kann.

Die in Fig. 11 und 12 dargestellten Phantomventil-Aktoren fahren "auf Anschlag". D.h., dass der Verschiebeweg des Stößels 55 durch die Anschläge 73 im Aufnahmeabschnitt 5 begrenzt ist. Unter der Abdichtungseinrichtung 1 ergibt sich somit vorzugsweise eine ebene Fläche.

Der Verschiebeweg der Stößel 55 kann jedoch auch unbegrenzt sein. Der Verschiebeweg kann in einer nicht dargestellten Ausführungsform nur durch die Verpressung der Abdichtungseinrichtung 1 im Ventilbereich gegen die externe Funktionseinrichtung 19 begrenzt sein. Auf diese Weise kann eine sichere Verpressung im Bereich der Ventile vorteilhaft sichergestellt werden.

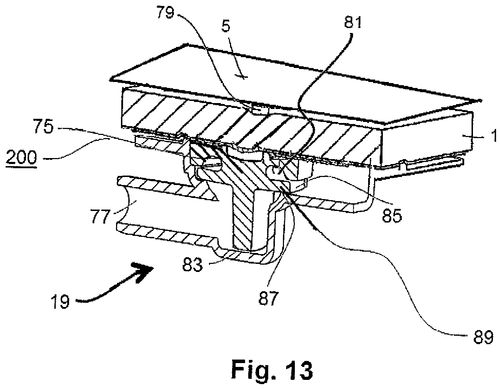

Fig. 13 zeigt schematisch vereinfacht einen Schnitt einer Ventileinrichtung 200 in einem Ventilzustand eines geschlossenen Rückschlagventils.

Die Ventileinrichtung 200 ist Teil einer externen Funktionseinrichtung 19 und weist einen Ventileinsatz 75 auf, welcher in einem Strömungskanal 77 der externen Funktionseinrichtung 19 angeordnet ist.

Wie in Fig. 13 gezeigt, ist die externe Funktionseinrichtung 19 zu ihrem Gebrauch derart an der Anordnung (hier nicht gezeigt) angeordnet, dass ein Bolzen 79 des Aufnahmeabschnitts 5 über einem oder in Wirkung auf einen Verformungsraum 81 der Ventileinrichtung 200 angeordnet ist.

Beim Ankoppeln der externen Funktionseinrichtung 19 an die Abdichtungseinrichtung 1 drückt der Bolzen 79 des Aufnahmeabschnitts 5, z.B. einer Aktor-Sensor-Platte, lokal gegen die Abdichtungseinrichtung 1, so dass diese in Richtung der externen Funktionseinrichtung 19 ausbeult und den Ventileinsatz 75 bis zu einem Anschlag 83 in das Hartteil der externen Funktionseinrichtung 19 drückt.

Ein elastischer Dichtring 85 des Ventileinsatzes 75 drückt in der dargestellten Rückschlagventilposition gegen einen starren Dichtring 87 des Hartteils der externen Funktionseinrichtung 19, so dass ein Spalt 89 geschlossen wird. Somit kann kein Fluid durch den Strömungskanal 77 der externen Funktionseinrichtung 19 strömen.

## Patentansprüche

1. Medizinische Anordnung (100) zur Behandlung wenigstens eines medizinischen Fluids, mit:
wenigstens einer Abdichteinrichtung (1) zum Abdichten wenigstens eines ersten Volumens der Anordnung (100) gegen wenigstens ein zweites Volumen, wobei das erste Volumen zum Aufnehmen einer externen Funktionseinrichtung (19) vorgesehen ist;
einem Stößel (55) zum Verformen der Abdichteinrichtung (1);
einem Aufnahmeabschnitt (5) zum Aufnehmen der externen Funktionseinrichtung (19);
einer Aktor-Membran (59);
einer Luftverteilerplatte (63) mit einer Öffnung (69) zum Zuführen von Gas in Richtung Stößel (55), in Richtung Aktor-Membran (59) oder in Richtung Abdichteinrichtung (1);
wobei der Aufnahmeabschnitt (5) eine Sacköffnung oder Bohrung (70) aufweist, in welcher der Stößel (55) axial bewegbar angeordnet ist; und
wobei die Aktor-Membran (59) ausgestaltet ist, um sich unter mittels des Gases ausgeübten Drucks auszudehnen und den Stößel (55) in Richtung Abdichteinrichtung (1) zu drücken.

2. Medizinische Anordnung (100) nach Anspruch 1, mit wenigstens einer ersten Verbindungseinrichtung (7), mittels welcher die Abdichtungseinrichtung (1) mit der Anordnung (100) verbindbar ist.

3. Medizinische Anordnung (100) nach Anspruch 2, wobei die erste Verbindungseinrichtung (7) zur kraft- oder formschlüssigen Verbindung mit einer zweiten Verbindungseinrichtung (9) ausgestaltet ist.

4. Medizinische Anordnung (100) nach einem der Ansprüche 2 bis 3, wobei die erste Verbindungseinrichtung (7) als einsteckbare Einrichtung ausgestaltet ist, welche in die als eine Aufnahmeeinrichtung für die einsteckbare Einrichtung ausgestaltete zweite Verbindungseinrichtung (9) einsteckbar ist.

5. Medizinische Anordnung (100) nach einem der Ansprüche 3 bis 4, wobei die zweite Verbindungseinrichtung (9) als einsteckbare Einrichtung ausgestaltet ist, welche in die als eine Aufnahmeeinrichtung für die einsteckbare Einrichtung ausgestaltete erste Verbindungseinrichtung (7) einsteckbar ist.

6. Medizinische Anordnung (100) nach einem der vorangegangenen Ansprüche, welche ferner eine dritte Verbindungseinrichtung (11) zur Verbindung der Abdichtungseinrichtung (1) mit einem Aufnahmeabschnitt (5) zum Aufnehmen der externen Funktionseinrichtung (19) aufweist.

7. Medizinische Anordnung (100) nach Anspruch 6, wobei die dritte Verbindungseinrichtung (11) ausgestaltet ist, um mit einer vierten Verbindungseinrichtung (13) des Aufnahmeabschnitts (5) zum Aufnehmen der externen Funktionseinrichtung (19) verbindbar zu sein.

8. Medizinische Anordnung (100) nach einem der Ansprüche 2 bis 7, wobei wenigstens die erste Verbindungseinrichtung (7), die zweite Verbindungseinrichtung (9), die dritte Verbindungseinrichtung (11) oder die vierte Verbindungseinrichtung (13) umlaufend ausgestaltet sind.

9. Medizinische Anordnung (100) nach einem der Ansprüche 3 bis 8, wobei wenigstens die erste Verbindungseinrichtung (7) zusammen mit der zweiten Verbindungseinrichtung (9), oder die dritte Verbindungseinrichtung (11) zusammen mit der vierten Verbindungseinrichtung (13) ausgestaltet sind, um jeweils gemeinsam eine fluiddichte Abdichtung zu bewirken.

10. Medizinische Anordnung (100) nach einem der Ansprüche 2 bis 9, wobei die erste Verbindungseinrichtung (7) in wenigstens einem Abschnitt (17) eine geringere Dicke aufweist als in einem weiteren Abschnitt der Abdichtungseinrichtung (1).

11. Medizinische Anordnung (100) nach einem der Ansprüche 6 bis 10, wobei die dritte Verbindungseinrichtung (11) oder die vierte Verbindungseinrichtung (13) wenigstens eine Noppe aufweist oder als Noppe ausgestaltet ist.

12. Medizinische Anordnung (100) nach einem der vorangegangenen Ansprüche, wobei die Abdichtungseinrichtung (1) ausgestaltet ist, um durch Aufbringen von Unterdruck mit der Oberfläche des Aufnahmeabschnitts (5) verbindbar zu sein.

13. Medizinische Anordnung (100) nach einem der vorangegangenen Ansprüche, wobei die Abdichtungseinrichtung (1) in wenigstens einem Abschnitt (17) vorverformte Bereiche aufweist, die durch Verschwächungen oder Verstärkungen einer Dicke der die vorverformten Bereiche umgebenden Bereiche ausgestaltet sind.

14. Medizinische Anordnung (100) nach einem der vorangegangenen Ansprüche, wobei die Abdichtungseinrichtung (1) wenigstens einen Sensor (41, 51), einen Aktor (54), eine Leitung (43) oder wenigstens einen Teil wenigstens eines derselben aufweist.

15. Medizinische Anordnung (100) nach einem der vorangegangenen Ansprüche, welche als Blutbehandlungsvorrichtung ausgebildet ist.

## Claims

1. A medical arrangement (100) for the treatment of at least one medical fluid comprising:
at least one sealing means (1) for sealing at least one first volume of the arrangement (100) against at least one second volume, wherein the first volume is provided for receiving at least one external functional means (19),
a tappet (55) for deforming the sealing means (1);
a reception portion (5) for receiving the external functional means (19),
an actuator diaphragm (59);
an air distribution plate (63) having an opening (69) for supplying gas in direction of the tappet (55), in direction of the actuator diaphragm (59) or in direction of the sealing means (1);
wherein the reception portion (5) comprises a bore or blind hole (70) wherein the tappet (55) is arranged inside the blind bore so as to be axially movable; and
wherein the actuator diaphragm (59) is configured to expand under the pressure exerted by means of the gas to push the tappet (55) in a direction toward the sealing means (1).

2. Medical arrangement (100) according to claim 1 having at least one first connection means (7) whereby the sealing means (1) may be connected to the arrangement (100).

3. Medical arrangement (100) according to claim 2, wherein the first connection means (7) is configured for frictional and/or form closure connection with a second connection means (9).

4. Medical arrangement (100) according to any one of the claims 2 to 3, wherein the first connection means (7) is configured as an insertable means and may be inserted into the second connection means(9) configured as a reception means for the insertable means.

5. Medical arrangement (100) according to any one of the claims 3 to 4, wherein the second connection means (9) is configured as an insertable means and may be inserted into the first connection means(7) configured as a reception means for the insertable means.

6. Medical arrangement (100) according to any one of the preceding claims, further including a third connection means (11) for connection of the sealing means (1) to a reception portion (5) for receiving the external functional means (19).

7. Medical arrangement (100) according to claim 6, wherein the third connection means (11) is configured for being connectable to a fourth connection means (13) of the reception portion (5) for receiving the external functional means (19).

8. Medical arrangement (100) according to any one of the claims 2 to 7, wherein at least the first connection means (7), the second connection means (9), the third connection means (11) or the fourth connection means (13) has a peripheral configuration.

9. Medical arrangement (100) according to any one of the claims 3 to 8, wherein the first connection means (7) together with the second connection means (9), or the third connection means (11) together with the fourth connection means (13), are configured so as to jointly produce a respective fluid-tight seal.

10. Medical arrangement (100) according to any one of the claims 2 to 9, wherein the first connection means (7) in at least one portion (17) has a lower thickness than in another portion of the sealing means (1).

11. Medical arrangement (100) according to any one of the claims 6 to 10, wherein the third connection means (11) or the fourth connection means (13) includes at least one knob or is configured as a knob.

12. Medical arrangement (100) according to any one of the preceding claims, wherein the sealing means (1) is configured for connection with the surface of the reception portion (5) by means of a negative pressure.

13. Medical arrangement (100) according to any one of the preceding claims, wherein the sealing means (1) in at least one portion (17) has predeformed areas which are configured by weakened sections or reinforcements of a thickness of the areas surrounding the predeformed areas.

14. Medical arrangement (100) according to any one of the preceding claims, wherein the sealing means (1) includes at least one sensor (41, 51), an actuator (54), a conduit (43) or at least one portion of at least one of these.

15. Medical arrangement (100) according to any one of the preceding claims configured as a blood treatment apparatus.

## Revendications

1. Dispositif médical (100) pour le traitement d'au moins un fluide médical comprenant :
au moins un dispositif d'étanchéité (1) pour étanchéifier au moins un premier volume du dispositif (100) par rapport à un second volume, où le premier volume est prévu pour recevoir une unité fonctionnelle externe (19),
un poussoir (55) prévu pour déformer le dispositif d'étanchéité (1) ;
un secteur de réception (5) pour recevoir l'unité fonctionnelle externe (19) ;
une membrane d'actionnement (59) ;
une plaque de distribution d'air (63) avec une ouverture (69) prévue pour introduire de l'air en direction du poussoir (55), en direction de la membrane d'actionnement (59) ou en direction du dispositif d'étanchéité (1) ;
où le secteur de réception (5) présente un trou borgne ou un perçage (70) dans lequel le poussoir (55) est agencé de façon à être mobile axialement ; et
où la membrane d'actionnement (59) est configurée pour se dilater sous la pression exercée par le gaz et pour pousser le poussoir (55) en direction du dispositif d' étanchéité (1).

2. Dispositif médical (100) selon la première revendication, avec au moins un premier moyen de liaison (7), au moyen duquel le dispositif d'étanchéité (1) peut être relié au dispositif (100).

3. Dispositif médical (100) selon la seconde revendication, où le premier moyen de liaison (7) est configuré pour être relié à un second moyen de liaison (9) par liaison complémentaire de force et/ou de forme.

4. Dispositif médical (100) selon l'une des revendications 2 à 3, où le premier moyen de liaison (7) est configuré comme dispositif enfichable, lequel est enfichable dans le second moyen de liaison (9) configuré comme unité de réception pour le dispositif enfichable.

5. Dispositif médical (100) selon l'une des revendications 3 à 4, où le second moyen de liaison (9) est configuré comme dispositif enfichable, lequel est enfichable dans le premier moyen de liaison (7) configuré comme unité de réception pour le dispositif enfichable.

6. Dispositif médical (100) selon l'une quelconque des revendications précédentes présentant de plus un troisième moyen de liaison (11) prévu pour relier le dispositif d'étanchéité (1) avec un secteur de réception (5) de l'unité fonctionnelle externe (19).

7. Dispositif médical (100) selon la revendication 6, où le troisième moyen de liaison (11) est configuré pour être relié à un quatrième moyen de liaison (13) du secteur de réception (5) prévu pour recevoir l'unité fonctionnelle externe (19).

8. Dispositif médical (100) selon l'une des revendications 2 à 7, où au moins le premier moyen de liaison (7), le second moyen de liaison (9), le troisième moyen de liaison (11) ou le quatrième moyen de liaison (13) est configuré de manière circonférentielle.

9. Dispositif médical (100) selon l'une des revendications 3 à 8, où au moins le premier moyen de liaison (7) conjointement avec le second moyen de liaison (9), ou le troisième moyen de liaison (11) conjointement avec le quatrième moyen de liaison (13) sont configurés de sorte à former ensemble, respectivement, un joint étanche au fluide.

10. dispositif médical (100) selon l'une des revendications 2 à 9, où le premier moyen de liaison (7) présente dans au moins un secteur (17) une épaisseur plus faible que dans un autre secteur du dispositif d'étanchéité (1).

11. Dispositif médical (100) selon l'une des revendications 6 à 10, où le troisième moyen de liaison (11) ou le quatrième moyen de liaison (13) présente au moins une nope ou un bouton ou est configuré comme une nope ou un bouton.

12. Dispositif médical (100) selon l'une quelconque des revendications précédentes, où le dispositif d'étanchéité (1) est configuré pour être relié à la surface du secteur de réception (5) au moyen d'une dépressurisation.

13. Dispositif médical (100) selon l'une quelconque des revendications précédentes, où le dispositif d'étanchéité (1) présente dans au moins un secteur (17) des régions prédéformées qui sont configurées comme un affaiblissement ou un renforcement de l'épaisseur des régions entourant les régions prédéformées.

14. Dispositif médical (100) selon l'une quelconque des revendications précédentes, où le dispositif d'étanchéité (1) présente au moins un capteur (41, 51), un actuateur (54), un conduit (43) ou au moins une partie d'au moins l'un d'entre eux.

15. Dispositif médical (100) selon l'une quelconque des revendications précédentes configuré comme appareil de traitement du sang.
